Europäisches Patentamt

European Patent Office

Office européen des brevets

(19)

(11) **EP 0 714 988 B1**

(12) **EUROPÄISCHE PATENTSCHRIFT**

(45) Veröffentlichungstag und Bekanntmachung des Hinweises auf die Patenterteilung:
**12.12.2001 Patentblatt 2001/50**

(51) Int Cl.7: **C12Q 1/68**, C07H 21/04, C12P 19/34, C12N 15/85, C07K 14/00, C12Q 1/70

(21) Anmeldenummer: **95890172.0**

(22) Anmeldetag: **26.09.1995**

(54) **Verfahren zur Quantifizierung von Nukleinsaüren**

Method for quantifying nucleic acids

Procédé pour quantifier d'acides nucléiques

(84) Benannte Vertragsstaaten:
**AT BE CH DE DK ES FR GB IE IT LI NL SE**

(30) Priorität: **26.09.1994 AT 183194**
**02.12.1994 AT 224594**

(43) Veröffentlichungstag der Anmeldung:
**05.06.1996 Patentblatt 1996/23**

(73) Patentinhaber: **Baxter Aktiengesellschaft**
**1221 Wien (AT)**

(72) Erfinder:
- **Falkner, Falko-Günter, Dr.**
 **A-2304 Orth/Donau (AT)**
- **Hämmerle, Thomas, Dr.**
 **A-2304 Orth/Donau (AT)**
- **Himmelspach, Michele, Dr.**
 **A-1100 Wien (AT)**
- **Kohl, Johann, Dr.**
 **A-1160 Wien (AT)**
- **Dorner, Friedrich, Prof. Dr.**
 **A-1230 Wien (AT)**

(74) Vertreter: **Weinzinger, Arnulf, Dipl.-Ing. et al**
**Patentanwälte**
**Sonn, Pawloy, Weinzinger & Köhler-Pavlik**
**Riemergasse 14**
**1010 Wien (AT)**

(56) Entgegenhaltungen:
EP-A- 0 521 318          WO-A-93/23573
WO-A-94/10342          WO-A-95/02067

- **JOURNAL OF VIROLOGICAL METHODS, Bd. 49, September 1994, Seiten 157-168, XP000600186 VAN GEMEN B ET AL: "A one tube quantitative HIV-1 RNA NASBA nucleic acid amplification assay using electrochemiluminescent (ECL) labelled probes"**
- **BIOTECHNIQUES, Bd. 14, Nr. 1, Januar 1993, Seiten 70-81, XP002022931 PIATAK M ET AL: "Quantitative competitive polymerase chain reaction for accurate determination of HIV DNA and RNA species"**

**Beschreibung**

Die Erfindung betrifft ein Verfahren zur Quantifizierung von Nukleinsäuren in einer Probe unter Anwendung von Nukleinsäure-Amplifizierung, wobei der Probe vor dem Amplifizierungsschritt eine gegebene Menge eines bekannten Nukleinsäuremoleküls als interner Standard zugegeben wird, welches Standard-Nukleinsäuremolekül sich von der zu quantifizierenden Nukleinsäure zumindest in einem detektierbaren Merkmal unterscheidet

Für die Diagnose von verschiedenen viralen Erkrankungen gibt es zur Zeit nur sehr unzuverlässige Detektionsmethoden. Eine Virusinfektion kann in einem frühen Stadium der Erkrankung oft nicht festgestellt werden, da die zur Verfügung stehenden ELISA-Methoden die wenigen im Blut zirkulierenden Virusproteine nicht detektieren können. Allerdings wäre es für Erfolge in der Therapie von unschätzbarem Wert, Infektionen möglichst früh zu erkennen. Weiters gilt für virale Infektionen auch, daß es im Verlaufe der Krankheit oder durch den Einfluß einer Therapie zu Schwankungen der Viruskonzentration im Blut kommt. Dabei kann die Anzahl der Viruspartikel so stark abnehmen, daß die herkömmlichen Methoden sich als zu wenig sensitiv erweisen. Falsch negative Ergebnisse durch ELISA-Test lassen in solchen Fällen den Krankheitsverlauf in einem falschen Bild erscheinen.

Ein anderer Aspekt bei der Detektion von Nukleinsäuren ist die Qualitätskontrolle von biotechnologisch hergestellten Produkten. Einerseits müssen Präparationen von immunogenen Virusproteinen, die aus infektiösen Viren gewonnen werden und als Impfstoffe eingesetzt werden sollen, auf ihren Gehalt an kontaminierender viraler Nukleinsäure hin überprüft werden. Andererseits werden rekombinante Produkte, die mit einem viralen Expressionssystem hergestellt wurden, auf kontaminierende Nukleinsäuren überprüft, die vom Expressionssystem stammen können. Die hier erlaubten Grenzwerte an kontaminierender Nukleinsäure sind von der Welt-Gesundheits-Organisation mit 100 pg pro Dosis, und von der U.S. Food and Drug Administration mit 10 pg pro Dosis festgelegt.

Die Entwicklung in der PCR-Technologie hat es ermöglicht, die Nachweisgrenze zur Detektion von Nukleinsäuren drastisch zu senken. Allerdings kämpft man vor allem bei niedrigen Nukleinsäurekonzentrationen nach wie vor mit großen Problemen in der Reproduzierbarkeit der Resultate. Das liegt einerseits an der noch nicht ausgereiften Technik, andererseits sind es oft nur wenige Kopien von Nukleinsäuren, die nachgewiesen werden sollen. Bei quantitativen Bestimmungen in diesem niedrigen Konzentrationsbereich wirken sich Fehler in der Analyse natürlich besonders drastisch aus.

Es ist dem Fachmann bestens bekannt, daß die Effizienz der PCR-Reaktion oftmals von Reaktionsgefäß zu Reaktionsgefäß unterschiedlich sein kann. Dieser Effizienzunterschied kann Unterschiede in den Ergebnissen von bis zu $10^5$ ergeben. Die Reproduzierbarkeit der mit den bekannten Methoden erhaltenen Quantifizierungsdaten ist daher immer noch nicht ausreichend.

Um die Reproduzierbarkeit der PCR-Reaktionen zu verbessern, wurde von Gilliland (PNAS 87:2725 (1990)) eine kompetitive PCR-Methode entwickelt. Zur Bestimmung von DNA-Mengen wird eine Verdünnungsreihe eines internen Standards verwendet, die gleichzeitig mit der Probe amplifiziert wird. Die PCR-Reaktion wird bis zur Sättigung durchgeführt. Dies erlaubt auch die Detektion der PCR-Produkte mittels Ethidiumbromidfärbung. Die PCR-Produkte werden anschliessend auf einem Gel aufgetrennt, die Kopienzahl der Probe mit der Kopienzahl der Standard-Verdünnungsreihe verglichen und so die Konzentration der Probe abgeschätzt. Diese Konzentrationsabschätzung ist dann exakt, wenn die Konzentration des Standards und der Probe etwa im Verhältnis 1:1 in einem Reaktionsgefäß amplifiziert wurden. Dies wiederum impliziert, daß die Bestimmung der DNA-Menge umso genauer erfolgt, je mehr Standardverdünnungen verwendet werden.

Eine Methode zur Quantifizierung von RNA wurde von Wang et al. (PNAS 86 (1989), 9717) vorgeschlagen. Diese PCR-Reaktion wird in der exponentiellen Phase gestoppt. Die Autoren schaffen sich durch Amplifikation unterschiedlicher Standardkonzentrationen eine Eichkurve. Da in der exponentiellen Reaktionsphase die Anzahl der PCR-Produkte direkt proportional zur Anzahl der PCR-Zyklen und zur Konzentration der RNA steigt, ist diese Eichkurve eine Gerade, in der man schließlich die Konzentration einer amplifizierten Probeablesen kann. Ein Nachteil dieser Methode ist, daß die Endkonzentration der PCR-Produkte relativ gering ist, sodaß man für die Detektion empfindliche Nachweismethoden anwenden muß. Wang et al. benutzen radioaktiv markierte Nukleotide.

Gemäß der WO 93/23573 kann durch kompetitive PCR die Konzentration viraler RNA im Bereich von $10^2$ bis $10^8$ Kopien bestimmt werden. Die Reproduzierbarkeit dieser Methode wird bei Mehrfachbestimmungen von HIV-1 mit einem Variationskoeffizienten von 0,26 ± 0,15 angegeben. Dabei werden die Bestimmungen umso genauer, je näher das Konzentrationsverhältnis von Probe zu Standard 1:1 liegt. Dabei ist zu berücksichtigen, daß bei dieser Methode die Standard-RNA erst nach der Probenaufbereitung zugegeben wird. Bei der Extraktion der Probe können aber bis zu 50 % der RNA verloren gehen. So wird schließlich die ursprünglich in der Probe enthaltene RNA-Menge auf den erst nach der Extraktion zugegebenen Standard bezogen. Dadurch wird lediglich die unterste Grenze der möglichen Kopienanzahl pro Probe bestimmt. Die durch die Extraktion verloren gegangenen Anteile können nicht berücksichtigt werden.

In der WO 94/20640 wird ebenfalls eine kompetitive RT-PCR-Methode beschrieben, von der behauptet wird, daß damit 100 Kopien des HIV-Genoms im Plasma von HIV-infizierten Patienten festgestellt werden kann. In Bereichen unter 100 Kopien erfolgt die Ermittlung der Konzentration der HIV-Genome nur mehr durch Abschätzung. Die Standardabweichung von Bestimmungen der Kopienanzahl im Bereich von $10^4$ bis $10^6$ Kopien liegt bei 22 %, wenn von dem gleichen Plasma eine Dreifachbestimmung gemacht wird; bei Doppelbestimmungen derselben RNA-Präparation liegt die Standardabweichung bei 15 % (WO 94/20640, Seite 22, Zeilen 8-13). Auch hier wird die Extraktionseffizienz der RNA-Extraktion nicht berücksichtigt. Die gefundenen Kopienzahlen der HIV RNA sind im Bereich von 4,4 x $10^3$ bis 9,3 x $10^6$. Nur bei einem einzigen Patienten werden 100 Kopien als "extrapolierter" Wert angegeben. Es stellt sich daher die Frage, ob diese Methode zur exakten Bestimmung von niedriger Kopienzahlen (unter $10^3$) überhaupt geeignet ist.

Eine Verbesserung in der Detektion von geringen Mengen an PCR-Produkten gelang Porcher et al. (BioTechniques 13 (1992), 106) durch den Einsatz von Fluoreszenz-markierten Primern und der Quantifizierung der PCR-Produkte mit einem automatischen Laser-Fluoreszenz-DNA-Sequencer.

Gemäß der WO95/02067 A1 werden zwei oder mehrere Plasmide als interne Standards zur Quantifizierung von DNA in einer Probe mittels PCR beschrieben. Gemäß diesem Verfahren wird zur Unterscheidung der verschiedenen Plasmide jeweils eine spezifische Sequenz von etwa 20 Nukleotiden in das jeweilige Plasmid insertiert. Dabei sollen die Längen der verschiedenen zu analysierenden Plasmide etwa gleich sein. Zur Unterscheidung der verschiedenen Sequenzen und ihrer Quantifizierung werden Detektionsproben eingesetzt, die spezifisch jeweils eine der Sequenzen in den Plasmiden erkennen. Es wird weiters angeführt, daß markierte Oligonukleotide, die spezifisch für jeweils eine der insertierten Sequenzen sind, zu den Proben mit den bereits amplifizierten Plasmiden zugesetzt werden. Durch spezifische Hybridisierung können anschließend die Plasmide quantifiziert werden. Es werden also zuerst die Plasmide amplifiziert und erst anschließend markierte Oligonukleotide zugesetzt.

Die EP 0 521 318 A2 betrifft cDNA-HCV-Polynukleotide, Polypeptide und Antikörper, die gegen die Polypeptide gerichtet sind.

Die vorliegende Erfindung stellt sich die Aufgabe, ein Verfahren zur Quantifizierung von Nukleinsäuren zur Verfügung zu stellen, welches eine sehr genaue und vor allem eine gut reproduzierbare Information bezüglich der Menge an Nukleinsäure in einer Probe ermöglicht und gleichzeitig Aussagen über die Nachweisgrenze der zu bestimmenden Nukleinsäure erlaubt.

Das erfindungsgemäße Verfahren der eingangs erwähnten Art ist dadurch gekennzeichnet, daß der Probe vor der Nukleinsäure-Amplifizierung bekannte Mengen von mindestens zwei sich zumindest in einem detektierbaren Merkmal voneinander und von der zu quantifizierenden Nukleinsäure unterscheidenden bekannten Nukleinsäuremolekülen als interner Standard zugegeben werden, die erhaltenen Mengen an amplifizierter Proben- und Standard-Nukleinsäure bestimmt werden und aus den erhaltenen Mengen die ursprünglich in der Probe vorhandene Menge an zu quantifizierender Nukleinsäure bestimmt wird und daß beim Amplifizieren Primer mit fluoreszierenden oder radioaktiven Gruppen oder chemischen Gruppen, die mit affinen Proteinen und nachgeschalteten Detektionsreaktionen detektiert werden können, vorzugsweise mit fluoreszierenden Gruppen, verwendet werden.

Das erfindungsgemäße Verfahren ermöglicht überraschenderweise eine sehr exakte und darüberhinaus gut reproduzierbare Quantifizierung von Nukleinsäuren aller Art.

Unter Nukleinsäure-Amplifizierung sind prinzipiell Verfahren zu verstehen, welche auf der von Mullis et al. (U.S. PS 4,683,195 und 4,683,202) und anderen entwickelten Technologie beruhen, beispielsweise die Polymerase-Kettenreaktion (PCR), die reverse Transkriptase-PCR (RT-PCR) oder die Ligase-CR (LCR).

Die Standard-Nukleinsäure muß sich in wenigstens einem detektierbaren Merkmal von der zu quantifizierenden Nukleinsäure unterscheiden, sie sollte aber mit Hilfe der gleichen Primer amplifiziert werden können. Als praktisch haben sich Standard-Nukleinsäuren erwiesen, die eine andere Größe als die zu quantifizierende Nukleinsäure oder eine unike Restriktionsschnittstelle aufweisen. Die Standard-Nukleinsäure ist bei Bestimmung von DNA-Mengen vorzugsweise eine DNA und bei Bestimmung von RNA-Mengen vorzugsweise eine RNA. Bevorzugte Standards unterscheiden sich von der zu quantifizierenden Nukleinsäure dadurch, daß deren PCR-Produkte sich in 1 % bis 20 % ihrer Länge bzw. durch mindestens 3, maximal 50 Nukleotide, unterscheiden. Eine Standardnukleinsäure, deren PCR-Produkt länger ist als das der zu bestimmenden Nukleinsäure, wird als "plus"("+")-Standard bezeichnet, und eine Standardnukleinsäure, deren PCR-Produkt kleiner ist als das der zu bestimmenden Nukleinsäure, wird als "minus"("-")-Standard bezeichnet. Die genaue Sequenz der Standard-Nukleinsäure sollte natürlich bekannt sein.

Die Standards werden im erfindungsgemäßen Verfahren bevorzugt in unterschiedlicher Konzentration eingesetzt, wobei einer der Standards in einer Konzentration knapp oberhalb der Nachweisgrenze zugegeben wird.

Die beim Amplifizieren verwendeten Primer enthalten Gruppen, welche die Nachweisgrenze der amplifizierten Nukleinsäuren erhöhen, beispielsweise fluoreszierende oder radioaktive Gruppen oder chemische Gruppen, die mit affinen Proteinen und nachgestalteten Detektionsreaktionen detektiert werden können (z.B. Biotin-Avidin, DIG-Markierung, etc.), wobei Primer mit fluoreszierenden Gruppen besonders bevorzugt sind.

Die Bestimmung der Nukleinsäure-Mengen (unter Nukleinsäure-Menge versteht man prinzipiell die Quantität an DNA oder RNA; eine Nukleinsäure-Menge kann z.B. in Form von Masse (mg, µg, ng, pg) oder als Anzahl der Kopien eines bestimmten Nukleinsäure-Moleküls angegeben werden) nach der Amplifizierung kann auf unterschiedlichste Art erfolgen, meist jedoch ist ein Schritt vorzusehen, bei welchem die amplifizierte Standard-Nukleinsäure von der amplifizierten, zu quantifizierenden Nukleinsäure getrennt wird und die getrennten Nukleinsäure-Mengen separat bestimmt werden. Vorzugsweise besteht dieser Trennungsschritt in einer Gelelektrophorese oder in einem chromatographisches Verfahren.

Als besonders geeignet haben sich Detektionsverfahren erwiesen, welche automatisch erfolgen und den Trennungs- und Quantifizierungsschritt kombinieren. Eine bevorzugte Ausführungsform des erfindungsgemäßen Verfahrens besteht daher darin, daß die Bestimmung der Mengen an amplifizierter Nukleinsäure unter Verwendung eines Nukleinsäure-Detektionsgerätes, vorzugsweise eines fluoreszenzempfindlichen Nukleinsäure-Detektionsgerätes, erfolgt. Beispiele für solche Nukleinsäure-Detektionsgeräte sind automatische DNA-Sequenzer mit laserinduzierten Fluoreszenz-Meßeinrichtungen (z.B. Gene Scanner® 373A der Firma Applied Biosystems), HPLC- oder Kapillar-Elektrophorese-System-Anlagen. Bei diesen Geräten ist es möglich, Nukleinsäure-Moleküle voneinander zu trennen, die sich lediglich um ein bp in der Länge unterscheiden.

Ein besonderer Vorteil des Gene-Scanner® ist es, unterschiedliche Fluoreszenzfarbstoffe in einer einzigen Spur unterscheiden zu können. Dies ermöglicht die gleichzeitige Aufarbeitung einer Vielzahl von Proben auf einem Gel, da alle am Gel zur Verfügung stehenden Spuren für Proben verwendet werden können. Weiters ist es möglich, eine Vielzahl von PCR-Produkten, markiert mit unterschiedlichen Fluoreszenz-Farbstoffen, in einer einzigen Bahn zu analysieren (Multiplex-PCR). Beim gleichzeitigen Nachweis von beispielsweise zwei verschiedenen Nukleinsäuren in einer Probe werden außerdem Aufwand und Kosten nahezu halbiert. Dies ist beim Einsatz des erfindungsgemäßen Verfahrens im Routinebetrieb von besonderem Vorteil, wenn beispielsweise eine Blutprobe auf HIV und HCV getestet werden soll. Im Gegensatz dazu kann der von Porcher et al. zur Analyse der PCR-Produkte verwendete automatische Laser-Fluoreszenz-DNA-Sequenzer nur einen Fluoreszenzfarbstoff (und damit nur eine DNA) pro Spur analysieren.

Eine bevorzugte Ausführungsform des erfindungsgemäßen Verfahrens betrifft daher ein Verfahren, bei dem mehrere erhaltene Mengen an amplifizierter Proben- und Standard-Nukleinsäuren in der gleichen Probe mittels der Multiplex-Analyse bestimmt werden.

Bei einer bevorzugten Ausführungsform des erfindungsgemäßen Verfahrens wird der Amplifizierungsschritt bereits in der exponentiellen Phase gestoppt.

Dadurch wird erreicht, daß das Verhältnis der Kopienanzahl der amplifizierten Standards direkt proportional zur Kopienanzahl der zu quantifizierenden Sequenz ist. Weiters kann durch Koamplifikation eines einzigen Standards die Kopienanzahl der zu bestimmenden Nukleinsäure festgestellt werden. In diesem Punkt ist die erfindungsgemäße Methode der oft verwendeten Methode von Gilliland et al. weit überlegen, da pro Probe lediglich eine Messung mit wenigstens zwei verschiedenen Standard-Molekülen durchgeführt werden muß, wogegen die Methode nach Gilliland um so genauer wird, je mehr Standards in verschiedenen Verdünnungen in verschiedenen Proben verwendet werden.

Ein besonders bevorzugtes Anwendungsgebiet des erfindungsgemäßen Verfahrens besteht in der Quantifizierung von Nukleinsäuren aus Mikroorganismen, vorzugsweise Nukleinsäuren aus HIV, Parvovirus, Herpesvirus, HAV, HBV, HCV, Baculovirus, Adenovirus, Influenzavirus, Vacciniavirus, Borreliaspezies, Salmonellaspezies oder Hefe. Diese Mikroorganismen sind sowohl als Pathogene als auch wegen ihrer Verwendung in der Herstellung von Impfstoffen und rekombinanten Proteinen interessant.

Im speziellen findet das erfindungsgemäße Verfahren Anwendung in der Quantifizierung viraler Nukleinsäuren, die als Kontaminationen in biotechnologisch hergestellten Produkten zurückbleiben können. Vorzugsweise kann es sich bei diesen biotechnologischen Produkten um immunogene Virusproteine oder Virusteile handeln, die durch biotechnologische Verfahren aus infektiösen Viren gewonnen werden; oder es handelt sich um rekombinant hergestellte Produkte, die mit einem viralen Expressionssystem hergestellt worden sind, und Kontaminationen der viralen Nukleinsäure des Expressionssystems aufweisen können.

Es werden vorzugsweise virale Nukleinsäuren in einer biologischen Probe, insbesondere in humanen Plasmen und deren Derivaten, gemäß dem erfindungsgemäßen Verfahren quantifiziert. Beispielsweise kann mit der vorliegenden Methodik der Verlauf einer Infektion oder die Überwachung von Impfungs- bzw. Therapiebehandlungen besser und genauer überwacht werden.

Dabei ist es von besonderer Bedeutung, daß mit dem erfindungsgemäßen Verfahren pathogene Viren in einer Konzentration bestimmt werden können, die mindestens eine Zehnerpotenz unterhalb der infektiösen Dosis dieser Viren liegt.

Um für die Nukleinsäurequantifizierung in der Probe einen möglichst genauen Wert zu erhalten, der nicht durch Manipulationen der zu untersuchenden Probe beeinflußt wird, setzt man gemäß einer bevorzugten Ausführungsform des erfindungsgemäßen Verfahrens die Standards direkt der Probe zu, vorzugsweise vor dem Extraktionsschritt, und unterzieht anschließend somit Proben-Nukleinsäuren und Standard-Nukleinsäuren den gleichen Probenaufbereitungsschritten (Koextraktion). Dies ermöglicht ein Quantifizierungsergebnis, das nicht nur erhöhte und unverfälschte Reproduzierbarkeit aufweist, sondern darüberhinaus auch unabhängig von Probenaufbereitungs-Manipulationen ist.

Ein weiterer Aspekt der vorliegenden Erfindung betrifft daher ein Verfahren zur Bestimmung der Nachweisgrenze von bestimmten Nukleinsäuren, bei welchem mindestens eine Standard-Nukleinsäure mit einer Konzentration von knapp oberhalb der Nachweisgrenze eingesetzt wird.

Vorzugsweise wird die Menge an Nukleinsäure in Kopienanzahl nach der folgenden Formel berechnet:

$$N_{Probe} = A_{Probe}/A_{Standard} \times N_{Standard} \times FxD$$

worin

$A_{Probe}$ die Peak-Fläche der amplifizierten Nukleinsäuren der Probe,
$A_{Standard}$ die Peak-Fläche des amplifizierten internen Standards darstellt,
$N_{Standard}$ die Zahl der eingesetzten Kopien des internen Standards,
F das Verhältnis des Einheitsvolumens zum extrahierten Volumen und
D der Verdünnungsfaktor (falls die Probe vor der Extraktion verdünnt worden ist), bedeutet

Für den besonderen Anwendungsbereich des erfindungsgemäßen Verfahrens, bei dem kontaminierende virale Nukleinsäuren quantifiziert werden sollen, wird, um den Forderungen der WHO und der FDA Genüge zu tun, die Menge an Nukleinsäure in pg/ml angegeben. Dazu kommt erfindungsgemäß folgende Formel zum Einsatz:

$$m_{Probe} = A_s/A_{st} \times N_{st} \times F1 \times D \times F2$$

worin

$A_S$ = die Peakfläche des PCR-Produkts der Probe,
$A_{st}$ = die Peakfläche des PCR-Produkts des Standards,
$N_{st}$ = die Kopienanzahl des Standard-Plasmids,
F1 = das Verhältnis des Einheitsvolumens zum extrahierten Volumen,
D = der Verdünnungsfaktor der Probe (falls notwendig) und
F2 = die Masse eines viralen Moleküls in pg (Molekulargewicht der viralen DNA/Avogadro-Zahl), ist.

Da nach dem erfindungsgemäßen Verfahren mindestens zwei Standards in unterschiedlichen Konzentrationen eingesetzt werden, erhält man nach der oben beschriebenen Berechnung mindestens zwei Werte für die Konzentration der Probe, aus denen man schließlich den Mittelwert berechnet. Im Routinebetrieb werden von jeder Probe in der Regel zwei Ansätze mit mindestens zwei Standards analysiert, wodurch schließlich ein Mittelwert aus vier Werten für die Konzen-tration der Probe gebildet werden kann.

Das erfindungsgemäße Verfahren zeichnet sich durch eine besonders hohe Reproduzierbarkeit aus. Die Standardabweichung, die bei Mehrfachbestimmungen erhalten wird, beträgt maximal 15 %, im allgemeinen aber meist unter 10 %, selbst wenn die Kopienzahl im niedrigen Konzentrationsbereich von $10^2$ liegt. Diese überaus geringen Standardabweichungen sind vor allem deswegen äußerst überraschend, da die jeweiligen Proben bei Mehrfachbestimmungen gemeinsam mit dem Standard auch jedesmal separat aufbereitet werden, d.h., daß auch Fehler in der Handhabung und Effizienz-unterschiede in der Extraktion der Probe und der PCR-Reaktion an sich in diese Standardabweichungen eingehen.

Die in den WO 93/23573 und WO 94/20640 beschriebenen Standardabweichungen von $0,26 \pm 0,15$ bzw. von 22 % bzw. 15 % wurden erhalten, indem die Probe nach der Aufbereitung in verschiedene Teile geteilt wird und diese Teile für die Bestimmung der Reproduzierbarkeit herangezogen werden. Dadurch gehen Effizienzunterschiede, die durch die Probenextraktion bzw. die RT-PCR auftreten können, nicht in die Bestimmung der Standardabweichung ein und es ergibt sich zwangsläufig eine bessere, wenn auch verfälschte Reproduzierbarkeit.

Beeinflußt wird die Effizienz der PCR-Reaktion zum Beispiel von der Art des zu amplifizierenden Nukleinsäure-Moleküls. Wird die erfindungsgemäße Methode zur Bestimmung von RNA-Viren herangezogen, so kämpft man mit dem allgemein bekannten Problem, daß die reverse Transkription unvollständig ist und lediglich wenige Prozent der vorhandenen RNA auch wirklich transkribiert werden. Weiters hat sich gezeigt, daß die Effizienz der Amplifikation für den "+"- bzw."-"-Standard in den einzelnen Bestimmungen differieren kann, im Mittel aber gleich ist. Dadurch kann es zu Schwankungen in den zu bestimmenden Konzentrationen kommen.

Gemäß einer bevorzugten Ausführungsform der Erfindung werden daher zur Vergrößerung der erhaltenen Information unterschiedliche Mengen der mindestens zwei Standard-Nukleinsäuren der Probe vor der Amplifizierung zugegeben werden.

Um weitere Ungenauigkeit in der Konzentrationsbestimmung der Probe auszuschließen, werden daher im Routineverfahren zwei Aliquote jeder Probe bestimmt, bei denen jeweils mindestens zwei Standards mitamplifiziert werden. So erhält man vier Meßwerte pro Probe, aus denen man sich dann einen Mittelwert errechnen kann. In den Beispielen ist die Genauigkeit und Reproduzierbarkeit der Methode gut demonstriert. Es wird auch gezeigt, daß die Methode über einen großen Konzentrationsbereich reproduzierbare Ergebnisse liefert.

Gemäß einer bevorzugten Ausführungsform des erfindungsgemäßen Verfahrens kommen zwei Standard-Nukleinsäuren zum Einsatz, welche eine gegenüber der zu quantifizierenden Nukleinsäure unterschiedliche Länge aufweisen, vorzugsweise eine Standard-Nukleinsäuresequenz, welche kürzer, und eine Standard-Nukleinsäuresequenz, welche länger ist als die zu quantifizierende Nukleinsäure. Ein besonders bevorzugter Längenunterschied liegt zwischen 1 % und 20 %.

Es hat sich für die erfindungsgemäße Methode von Vorteil erwiesen, die Nukleinsäure der internen Standards in linearisierter Form der PCR-Reaktion zuzusetzen. Dadurch werden weitere Unterschiede in der Effizienz der Reaktion ausgeglichen, die auf die unterschiedliche Form der zu amplifizierenden Nukleinsäure zurückzuführen sind.

Wichtige Kriterien bei der Quantifizierung von Nukleinsäuren sind - wie erwähnt - die Sensitivität und die Reproduzierbarkeit. Mit dem erfindungsgemäßen Verfahren können Nukleinsäure-Mengen im Bereich von 1 bis 500 Kopien wesentlich präziser und reproduzierbarer als mit den im Stand der Technik beschriebenen Methoden bestimmt werden. Damit ist aber keineswegs die Reproduzierbarkeitsgrenze der Methode erreicht.

Das Verwendungsspektrum des erfindungsgemäßen Verfahrens umfaßt beispielsweise die qualitative und quantitative Analyse von biologischen Proben auf Nukleinsäuren, insbesondere Blut und Blutderivate und biotechnologische Produkte. Eine weitere beispielhafte Einsatzmöglichkeit liegt in der Diagnose und/oder der Überwachung des Verlaufes von Infektionen sowie in der Überwachung von Impf- und Therapiebehandlungen.

Gemäß einem weiteren Aspekt betrifft die vorliegende Erfindung auch die Verwendung des erfindungsgemäßen Verfahrens zur Detektion von Nukleinsäuren in biologischen Proben.

Im weiteren kämpft die Qualitätskontrolle insbesondere bei Impfstoffen oder biotechnologisch erzeugten Proteinen mit Background-Problemen. So kann man beim Bestimmen von kontaminierender Nukleinsäure (chromosomale DNA, RNA, virale DNA und RNA) bei Primaten-Zellkulturen auch die Verunreinigungen durch die Handhabung während der Produktion oder während der Aufarbeitung der Produkte durch das erfindungsgemäße Verfahren erfassen, wenn die eingesetzten Primer spezifisch sind. Erfolgt die Produktion von rekombinanten Proteinen allerdings in Nicht-Primaten-Zellkulturen, wie zum Beispiel CHO (Chinese Hamster Ovarien), BHK (Baby Hamster Nierenzellen) oder CEC (Hühner-Embryozellen), so ist die Nachweisgrenze mit der erfindungsgemäßen Methode weit niedriger, da das Problem der Verunreinigungen durch die Handhabung der Probe wegfällt. Besonders bevorzugt wird die erfindungsgemäße Quantifizierungsmetho-dik bei Nukleinsäuren aus CHO-, Vero-(Affenzellinie), BHK-, SK-Hepl-(menschliche Leberzellinie), Hybridom- oder CEC-Zellen angewendet, da diese Zellkulturen am gebräuchlichsten sind bei der Produktion von Impfstoffen oder biotechnologisch hergestellten Proteinen.

Die Auswahl der Primerpaare ist selbstverständlich ebenfalls ein wichtiger Faktor, um eine gute Quantifizierung zu erhalten. Daher betrifft die vorliegende Erfindung gemäß einem weiteren Aspekt Primer, welche im vorliegenden Verfahren zur Anwendung kommen, nämlich

```
HAV+2058:ACTGCCATTGGGAAGCTTATTGTG      HAV 2058-2081  Seq.ID 3,


HAV-2172:CATCCATAGCATGATAAAGAGGAGC    HAV 2196-2172  Seq.ID 4,
(Numerierung nach Cohen et al. (J.Virol.61 ( 987) 50-59)


HCV32EXT:   CTGTGAGGAACTACTGTCTTACGCAG   HCV 45-70   Seq.ID 5,


HCVPT4: CGGTTCCGCAGACCACTATG  HCV  158-139 Seq.ID 6,
(Numerierung nach Han et al. (PNAS 88 (1991) 1711-1715)


HBV+1780B:CATTGATCCTTATAAAGAATTTGGAGC HBV 1780-1806  Seq.ID 7
```

und

```
HBV-1960B:CCAGCAGAGAATTGCTTGCCTGAG      HBV 1983-1960   Seq.ID 8
(Numerierung nach Fujiyama et al. (Nucleic Acids Res.11 (1983)
4601-4610)


opr1-r: AAA ATA GGA TCA TGA TGG C Vaccinia 84625-84644 Seq.ID 9,


opr1-f:ATA TTA GAT GGT GCC ACC GT Vaccinia 84761-84743 Seq.ID 10
(Numerierung nach Goebel et al., Virology 179: 247-266, 1990),


gD1/B:   AACTACCCGATCATCAG   HSV 138364-138381 Seq.ID 11,


gD2R/B: AGGCCCACTATGACGACA      HSV 138477-138456  Seq.ID 12
(McGeoch D.J., et al., EMBL GenBank ID HE1CG).
```

Gemäß einem weiteren Aspekt betrifft die vorliegende Erfindung die Verwendung eines Primers mit der Sequenz gemäß der Seq.ID6 in einem der oben beschriebenen erfindungsgemäßen Verfahren.

Weiters betrifft die vorliegende Erfindung Plasmide für die Herstellung der Standards, nämlich

pgag1 (bestehend aus dem bekannten pBS/SK -Plasmid und einem Insert zwischen der Pst I und der Apa I Stelle der multiplen Klonierungsstelle, welches Insert die Basenpaare (bp) 1417 bis 2008 der HIV-1 Sequenz aus Ratner et al. (Nature 313 (1985), 277-284) enthält),

pgag-15 (abgeleitet aus pgag1 mit einer Deletion von 15 bp ab bp 1593 der HIV-1 Sequenz aus Ratner et al.),

pgag+12 (abgeleitet aus pgag1, indem eine 12 Nukleotide lange Insertion an der bp1593-Stelle eingefügt wurde),

pHAV-wt (bestehend aus dem bekannten pCRII-Plasmid) und einem Insert an der multiplen Klonierungsstelle des pCRII-Plasmids, welches Insert die bp 2020 bis 2226 der cDNA-Sequenz aus Cohen et al. enthält),

pHAV-10bp (abgeleitet aus pHAV-wt mit einer Deletion von 10 bp ab bp 2100 der HAV-Sequenz aus Cohen et al. eingefügt wurde),

pHAV+9bp (abgeleitet aus pHAV-wt, indem eine 9 Nukleotide lange Insertion an der bp2100-Stelle eingefügt wurde),

pHCV-wt (bestehend aus dem bekannten pBS/SK—Plasmid und einem Insert an der EcoRV-Stelle diese Plasmids, welches Insert die bp27 bis 313 der cDNA-Sequenz aus Han et al. enthält),

pHCV-7bp (abgeleitet aus pHCV-wt mit einer Deletion von 7bp ab bp 126 der HCV-Sequenz aus Han et al. eingefügt wurde),

pHCV+8bp (abgeleitet aus pHCV-wt, indem eine 8 Nukleotide lange Insertion an der bp126-Stelle eingefügt wurde),

pHBV-wt (bestehend aus dem bekannten pCRII-Plasmid und einem Insert, welches Insert die bp 1763 bis 2032 des HBV-Genoms gemäß Fujiyama et al. enthält),

pHBV-9bp (abgeleitet aus pHBV-wt mit einer Deletion von 9bp ab bp 1868 der HBV-Sequenz aus Fujiyama et al. eingefügt wurde),

pHBV+12bp (abgeleitet aus pHBV-wt, indem eine 12 Nukleotide lange Insertion an der bp1868-Stelle eingefügt wurde),

pVV-wt (abgeleitet aus dem bekannten pTZ19R-Plasmid von Pharmacia mit einem Insert zwischen den beiden PvuII-Stellen dieses Plasmids, welches Insert die bp 83855 bis 84761 des Thymidinkinase-Gens des Vaccinia-Virus (nach Goebel et al., 1990) enthält.),

pVV-21 (abgeleitet aus pVV-wt mit einer Deletion von 21 Nukleotiden (bp 84718 bis 84738),

pVV+24 (abgeleitet aus pVV-wt mit einer Insertion von 24 bp an der Stelle 84713),

pHerp (abgeleitet von dem bekannten pCRII-Plasmid (Firma In Vitrogen) und einem Insert, welches Insert die bp 138364 bis 138784 des HSV-Genoms gemäß McGeoch et al., EMBL GenBank DE 1 CG1 enthält),

pHerp-9 (abgeleitet von p-Herp mit einer Deletion von 9 Nukleotiden (bp 138388 bis 138396) und

pHerp+10 (abgeleitet von p-Herp mit einer Insertion von 10 bp bei bp 138407.

Gemäß einem weiteren Aspekt bezieht sich die vorliegende Erfindung auch auf ein Set zur Quantifizierung von Nukleinsäuren in einer Probe, welches umfaßt:

- mindestens zwei bekannte Nukleinsäuren als interne Standards, welches sich voneinander und von den zu quantifizierenden Nukleinsäuren in mindestens einem nachweisbaren Charakteristikum unterscheiden,
- Fluoreszenz-markierte Primer, die an die Standard-Nukleinsäure und an die zu quantifizierende Nukleinsäure binden,
- positive Kontrollen, welche bekannte Mengen einer Nukleinsäure, an der Interesse besteht, aufweisen,
- eine negative Kontrolle, welche Humanplasma, das frei von der viralen Nukleinsäure ist, an der Interesse besteht, aufweist, und
- eine Arbeitsanleitung.

Bevorzugte Ausführungsformen des Sets gemäß der vorliegenden Erfindung sind die folgenden:

1.) Set zur Quantifizierung von HIV-RNA in einer Probe, welches umfaßt:

- mindestens zwei interne Standards, die eine in vitro-transkribierte RNA aufweisen, welche vom pBS/SK-Plasmid mit einem Insert in der multiplen Klonierungsstelle, welches die bp1417-2008 des HIV I enthält, wobei die bp1593-1607 deletiert sind, [pgag-15] und vom pBS/SK-Plasmid mit einem Insert in der mltiplen Klonierungsstelle, welches die bp1417-2008 des HIV I enthält, wobei ein 12bp langes Insert an der Stelle 1593 eingefügt ist, [pgag+12] stammt,
- die Fluoreszenz-markierten Primer Seq.ID 1 (SK38) und Seq.ID 2 (SK39)
- eine positive Kontrolle, welche bekannte Mengen an HIV-1-Partikel aufweist,
- eine negative Kontrolle, welche Humanplasma aufweist, das frei von Virus-Nukleinsäuren ist und
- eine Arbeitsanleitung.

2.) Set zur Quantifizierung von HCV-RNA in einer Probe, welches aufweist:

- mindestens zwei interne Standards, die eine in vitro-transkribierte RNA aufweisen, welche vom pBS/SK-Plasmid mit einem Insert in der EcoRV-Stelle, welches die bp27 bis 313 der cDNA-HCV-Sequenz enthält, wobei die bp126 bis 132 deletiert sind, [pHCV-7bp] und vom pBS/SK-Plasmid mit einem Insert in der EcoRV-Stelle, welches die bp27 bis 313 der cDNA-HCV-Sequenz enthält, wobei ein 8bp langes Insert an der Stelle 126 eingefügt ist, [pHCV+8bp] stammt,
- die Fluoreszenz-markierten Primer Seq.ID 5 (HCV32Ext) und Seq.ID 6 (HCVPT4)
- positive Kontrollen, welche bekannte Mengen an HCV-Partikel aufweisen,
- eine negative Kontrolle, welche Humanplasma aufweist, das frei von Virus-Nukleinsäuren ist und
- eine Arbeitsanleitung.

3.) Set zur Quantifizierung von HAV-RNA in einer Probe, welches aufweist:

- mindestens zwei interne Standards, die eine in vitro-transkribierte RNA aufweisen, welche vom pCRII-Plasmid mit einem Insert in der multipen Klonierungsstelle, welches die bp2020 bis 2226 der cDNA-HAV-Sequenz enthält, wobei die bp2100 bis 2109 der HAV-Sequenz deletiert sind, [pHAV-10bp] und vom pCRII-Plasmid mit einem Insert in der multiplen Klonierungsstelle, welches die bp2020 bis 2226 der cDNA-HAV-Sequenz enthält, wobei ein 9 bp langes Insert an der Stelle 2100 eingefügt ist, [pHAV+9bp] stammt,

- die Fluoreszenz-markierten Primer Seq.ID 3 (HAV+2058) und Seq.ID 4 (HAV-2172)
- positive Kontrollen, welche bekannte Mengen an HAV-Partikel aufweisen,
- eine negative Kontrolle, welche Humanplasma aufweist, das frei von Virus-Nukleinsäuren ist und
- eine Arbeitsanleitung.

4.) Set zur Quantifizierung von HBV-DNA in einer Probe, welches aufweist:

- mindestens zwei interne Standards, die pCRII-Plasmid mit einem Insert, welches die bp1763 bis 2032 des HBV enthält, wobei die bp 1868 bis 1876 deletiert sind, [pHBV-9bp] und das PCRII-Plasmid mit einem Insert, welches die bp1763 bis 2032 des HBV enthält, wobei ein 12 bp langes Insert an der Stelle 1868 eingefügt ist, [pHBV+12bp] aufweisen,
- die Fluoreszenz-markierten Primer Seq.ID 7 (HBV+1780B) und Seq.ID 8 (HBV-1960B)
- positive Kontrollen, welche bekannte Mengen an HBV-Partikel aufweisen,
- eine negative Kontrolle, welche Humanplasma aufweist, das frei von Virus-Nukleinsäuren ist und
- eine Arbeitsanleitung.

5.) Set zur Quantifizierung von HSV-DNA in einer Probe, welches aufweist:

- als interne Standards, die das pCRII-Plasmid mit einem Insert, welches die bp138364 bis 138784 des HSV enthält, wobei die bp138388 bis 138396 deletiert sind, [pHerp-9] und das pCRII-Plasmid mit einem Insert, welches die 138364 bis 138784 des HSV enthält, wobei ein 10bp langes Insert an der Stelle 138407 eingefügt ist [pHerp+10] aufweisen,
- die Fluoreszenz-markierten Primer Seq.ID 11 (gDR/B) und Seq.ID 12 (gDR2R/B)
- positive Kontrollen, welche bekannte Mengen an HSV-Partikel aufweisen,
- eine negative Kontrolle, welche Humanplasma aufweist, das frei von Virus-Nukleinsäuren ist und
- eine Arbeitsanleitung.

Die Erfindung wird in den nachstehenden Beispielen und den dazugehörigen Zeichnungsfiguren, auf die sie jedoch nicht beschränkt sein soll, noch weiter erläutert. Insbesondere wird in den Beispielen gezeigt, daß das erfindungsgemäße Verfahren sich hervorragend für eine routinemäßige, schnelle und trotzdem genaue und reproduzierbare Quantifizierung von Nukleinsäuren in unterschiedlichsten Proben eignet.

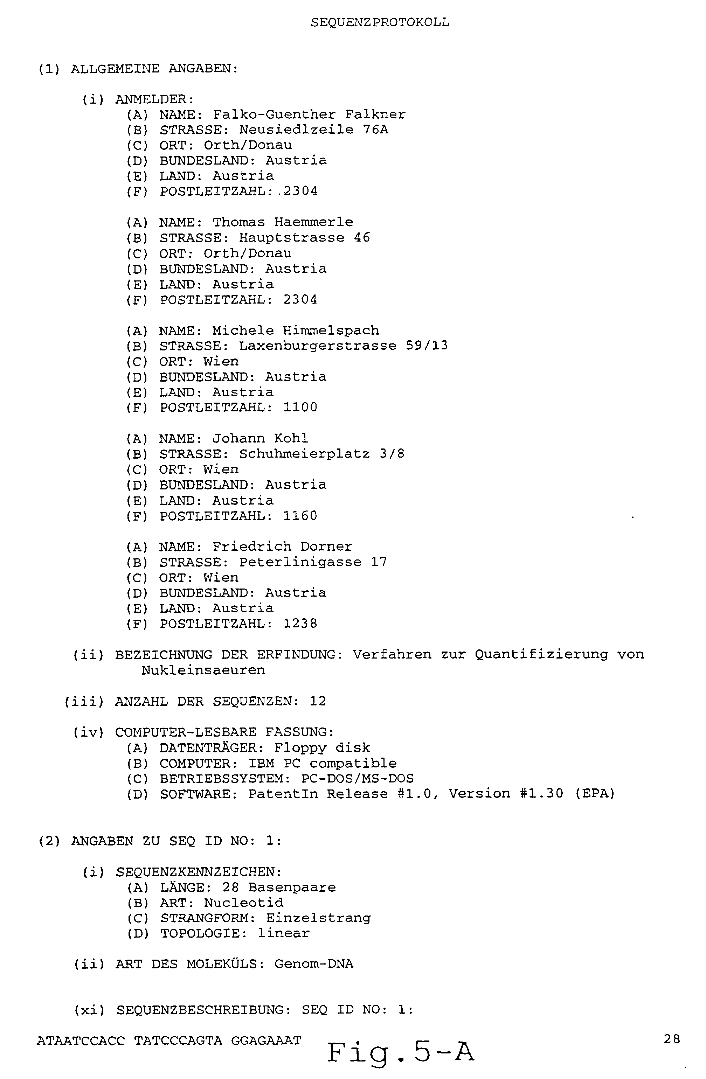

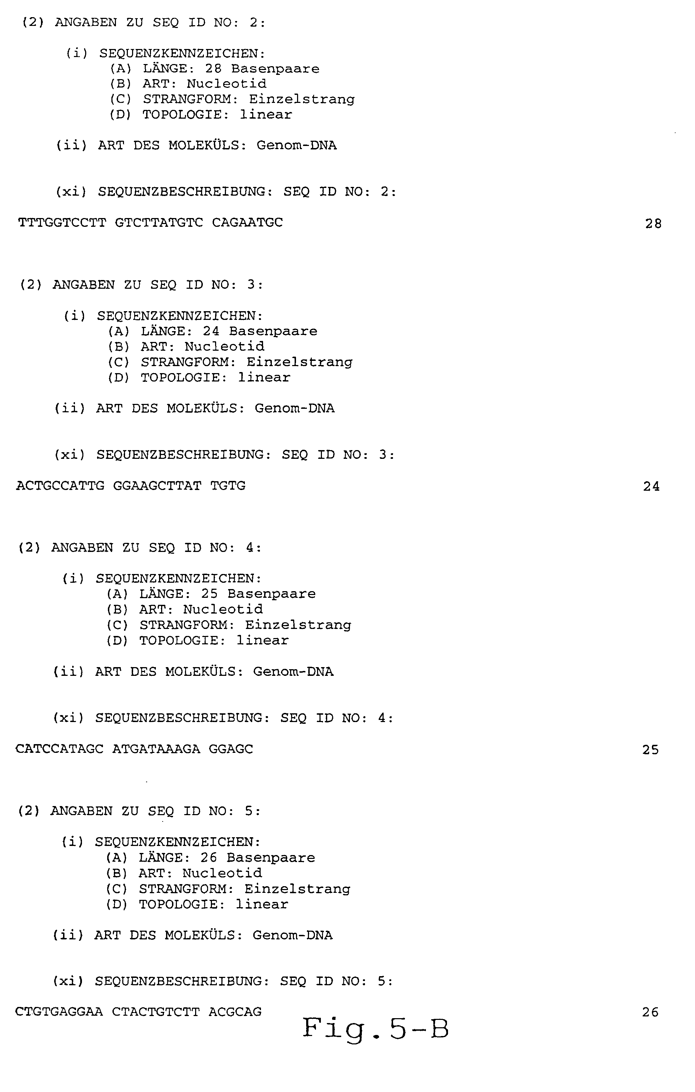

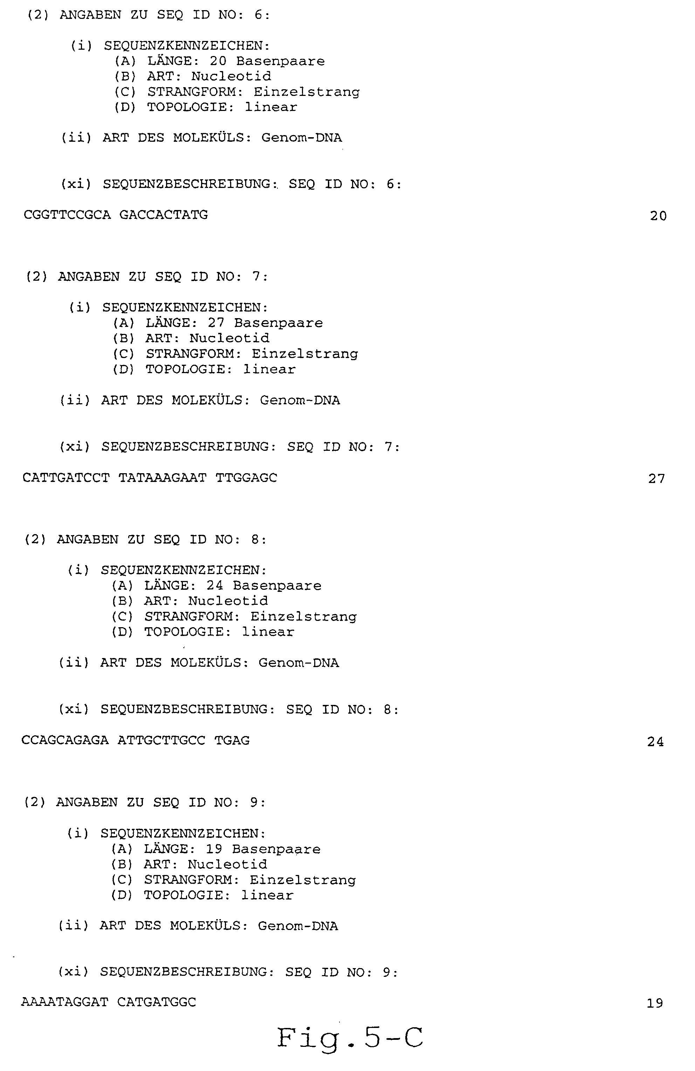

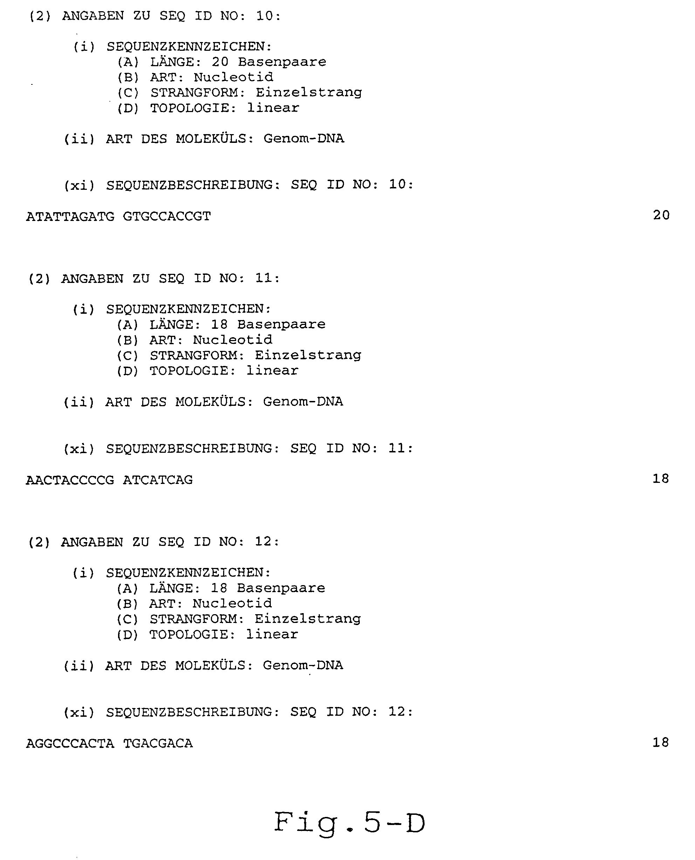

Es zeigen: Fig.1 die Klonierung von pgag-15 und pgag+12; Fig.2 die Ergebnisse der Quantifizierung von HIV mit RT-PCR; Fig.3 die Ergebnisse der Quantifizierung von HAV mit RT-PCR; Fig. 4 die Ergebnisse der Quantifizierung von HBV; und Fig.5 ist das Sequenzprotokoll.

Beispiele

1. Allgemeine Arbeitsvorschriften:

1.1. Prinzip des Verfahrens

Nukleinsäuren unterschiedlicher Herkunft werden mittels PCR unter Verwendung von Primern, welche fluoreszierende Gruppen haben, amplifiziert (Saiki et al., Science 239 (1988) 487-491). Die Analyse und die Quantifizierung der erhaltenen amplifizierten PCR-Produkte wurde mit Hilfe eines automatischen DNA-Sequenzierers mit laserinduzierter Fluoreszenz-Meßeinrichtung (DNA-Sequenzierer 373A mit Gene Scan® -Software von Applied Biosystems) ausgeführt. Dieses Instrument ist in der Lage, die Fluoreszenz-markierten PCR-Produkte mittels einer Gelelektrophorese in einem Polyacrylamidgel unter denaturierenden Bedingungen der Größe nach aufzutrennen und deren Menge quantitativ zu bestimmen. Die Kopienzahl bestimmter Sequenzen in der Probe wird auf Grundlage der erhaltenen Intensitäten der PCR-Produkte von zu quantifizierender Nukleinsäure und mindestens zwei internen Standards bestimmt.

1.2.1. Extraktion der DNA viraler Partikel

500 µl der Probe werden für 20 min bei 70000 rpm in einer Ultrazentrifuge zentrifugiert. Das Pellet wird in 500 µl 10 mM TRIS/HCl pH 8,0 und 10 µl Proteinase K (Boehringer Mannheim, 20 mg/ml), sowie 10 µl 20% SDS aufgelöst. Eine bestimmte Menge an Standard-Nukleinsäure und 1 µg Hering-Sperma-DNA werden zugesetzt, und die Probe wird 1 h lang bei 56°C inkubiert. Die Probe wird nacheinander mit Phenol und Chloroform extrahiert, und 10 µl Glykogen (Boehringer Mannheim, 20 mg/ml) werden zugesetzt. Anschließend wird mit Ethanol präzipitiert, zentrifugiert, das Pellet gewaschen und schließlich in Wasser wieder gelöst.

### 1.2.2. Extraktion proviraler DNA

5 x $10^5$ Zellen werden in 100 µl Lysis-Puffer (1 X PCR-Puffer von Boehringer, 0,5 mg/ml Proteinase K, 0,45 % Tween) 5 h bei 56°C lysiert. Aliquote davon werden für die PCR eingesetzt.

### 1.2.3. Extraktion viraler Rest-DNA

500 µl der Probe werden in 5 µl 10 mM TRIS/HCl pH 8,0 und 10 µl Proteinase K (Boehringer Mannheim, 20 mg/ml) aufgelöst. Nach Inkubation über Nacht bei 37°C oder für 4 h bei 56°C wird eine bestimmte Menge an Standard-Nukleinsäure zugesetzt, die Probe nacheinander mit Phenol und Chloroform extrahiert und 10 µl Glykogen (Boehringer Mannheim, 20 mg/ml) zugesetzt. Anschließend wird mit Ethanol präzipitiert, zentrifugiert, das Pellet gewaschen und schließlich in Wasser wieder gelöst.

### 1.2.4. Extraktion von RNA

1 ml Plasma bzw. mit PBS verdünntes Plasma wird bei 70000 rpm 20 min. zentrifugiert. Der Überstand wird durch Absaugen entfernt. Das Pellet wird in 1 ml Guanidiumisothiocyanat-Lösung (RNAzol® der Firma Biotexc) aufgenommen und 5 µl 1 mg/ml t-RNA aus Hefe und eine vorbestimmte Menge, z.B. 20 µl, Standard-RNA zugegeben.

Es werden eine vorbestimmte Anzahl, z.B. 400 und 1200 Kopien, des Minus- und Plus-RNA-Standards zugegeben und gevortext. Die Lösung wird 10 min bei 70°C erhitzt, dann 1/10 Volumen Chloro form zugegeben und für 10 min auf Eis inkubiert. Dann wird für 5 min in einer Tischzentrifuge zentrifugiert, der Überstand in neue Röhrchen transferiert. 500 µl Isopropanol wird zugegeben und 15 min auf -80°C gestellt. Anschließend wird 10 min zentrifugiert, 2 x mit 70 % Ethanol gewaschen und das Pellet in 50 µl Wasser aufgenommen. Für die RT-PCR-Reaktion werden 5 µl eingesetzt.

### 1.3.1. PCR für den Nachweis von DNA

Der PCR-Ansatz enthält in bekannter Weise ein Aliquot der extrahierten Nukleinsäure, PCR-Puffer (Boehringer Mannheim), $MgCl_2$, dNTPs, Primer, Taq-DNA-Polymerase (Boehringer Mannheim, 5,0 E/µl) und Wasser. Die PCR wird gemäß den Angaben des Herstellers von Puffer und Enzym bzw. gemäß üblicher Arbeitsvorschriften (Mulliset al., Methods in Enzymology 155 (1987), 335) in einem PCR-Apparatur (GeneAmp PCR System 9600 der Firma Perkin-Elmer) durchgeführt.

### 1.3.2. RT-PCR für den Nachweis von RNA:

Der RT-PCR-Ansatz enthält in bekannter Weise ein Aliquot der extrahierten Nukleinsäure RT-Puffer von Perkin-Elmer, $MgCl_2$, dNTPs, den RT-Primer und rT.th.-Polymerase (Perkin-Elmer, 2,5 E/µl) und Wasser. Die RT wird gemäß den Angaben des Herstellers von Puffer und Enzym bzw. gemäß üblicher Arbeitsvorschriften (Mullis et al., Methods in Enzymology 155 (1987), 335) in einem PCR-Apparatur (GeneAmp PCR System 9600 der Firma Perkin-Elmer) durchgeführt.

Für die PCR-Reaktion werden noch $MgCl_2$, ein Chelatpuffer und der zweite Primer zugegeben. Dann wird die PCR nach den oben beschriebenen Angaben durchgeführt.

### 1.4. Analyse der Produkte

Für die Bestimmung und Quantifizierung der PCR-Produkte werden der PCR-Lösung 0,5 bis 1,0 µl entnommen und in einem 373A Instrument der Firma Applied Biosystems gemäß den Angaben des Herstellers analysiert.

### 1.5. Anordnung der Kontrollen in (RT)-PCR

Zusätzlich zu strengen internen Kontrollen sichert eine Anordnung von positiven und negativen Kontrollen die PCR-Ergebnisse. In den verschiedenen Kontrollen werden die Standards in verschiedenen Schritten des Analyse-Verfahrens zugegeben (Liste 1).

| Liste 1. Anordnung der Kontrollen in (RT)-PCR | |
|---|---|
| Art | Kommentar |
| | negative Kontrollen |
| k1** | Reagenz-Leer-Kontrolle. Zugabe von Wasser zur RT-Mischung und PCR-Mischung. Die Handhabung erfolgt in dem (Labor-) Bereich, in welchem die Stammlösungen gelagert werden. Die Probe wird bis zur PCR auf Eis gehalten. Diese Kontrolle liefert Informationen über die Verunreinigung in den Reagenzien. |
| k3** | Verunreinigungskontrolle. Zugabe von Wasser anstelle von Extrakt zur RT-Mischung. Die Analyse erfolgt wie für die anderen Proben. Diese Kontrolle liefert Informationen über die Verunreinigung aufgrund der Handhabung in jenem (Labor-)Bereich, in welchem die RT-PCR durchgeführt wird. |
| k4* | Verunreinigungskontrolle. Analyse eines Extrakts, hergestellt aus einem negativen Kontrollplasma oder PBS. Mit dieser Kontrolle werden infolge des Extraktionsvorganges falsch positive Ergebnisse nachgewiesen. Keine Zugabe interner Standards. |
| | positive Kontrollen: |
| k5* | Interner Standard/Reagenz-Kontrolle. Direkte Zugabe des internen Standard in die RT-PCR/ PCR-Reaktion ohne vorherige Extraktion. Mit dieser Kontrolle wird die Integrität der internen Standards und die Funktionalität des Reagenz getestet. |
| k6*** | Verifizierungskontrolle. Analyse einer bestimmten Menge eines Verifizierers, hergeleitet aus Wildtyp-Standard-Plasmiden durch in vitro-Transkription. Mit dieser Kontrolle wird der Extraktionsvorgang und die Menge der internen Standards getestet. |
| k7* | Virusteilchenkontrolle. Analyse eines eine bestimmte Menge an Virusteilchen enthaltenden Aliquots, das in negativem Plasma oder PBS auf eine geeignete Konzentration verdünnt wurde. Mit dieser Kontrolle wird die Gesamtleistung der verwendeten Untersuchung getestet. |

* die Kontrollen k4, k5 und k7 werden in jeder Extraktionsreihe durchgeführt;

** die Kontrollen k1 und k3 werden durchgeführt, sobald falsch positive Wildtyp-Signale auftreten;

*** k6 wird nur durchgeführt, wenn neue Chargen interner Standards hergestellt werden; die Kontrolle k2 wurde seit der Einführung der hier beschriebenen Vorgangsweise mit einem Enzym in nur einem Röhrchen ausgelassen.

2. Beispiel 1: Quantifizierung von HIV durch reverse Transkriptase-PCR (RT-PCR)

Bei dieser Quantifizierung werden Primer verwendet, welche in den cDNA-Sequenzen des HIV-1 binden und durch RT-PCR von Wildtyp-RNA ein 115 bp großes Produkt ergeben, nämlich

```
    SK38:    ATAATCCACCTATCCCAGTAGGAGAAAT HIV-1 1551-1578 Seq.ID 1

    SK39:    TTTGGTCCTTGTCTTATGTCCAGAATGC HIV-1 1665-1638 Seq.ID 2
```

(Numerierung nach Ratner et al.). Die Primer wurden unter Verwendung der Phosphoamidit-Chemie auf einem DNA-Synthesizer hergestellt (Applied Biosystems 394 DNA Synthesizer).

Die Standard-Plasmide pgag-15 und pgag+12 sind abgeleitet aus dem Plasmid pgag1, welches aus dem bekannten pBS/SK⁻-Plasmid (Firma Stratagene) und einem Insert in der multiplen Klonierungsstelle dieses Plasmids besteht, welches Insert die bp 1417 bis 2008 des HIV-1 aus Ratner et al. enthält.

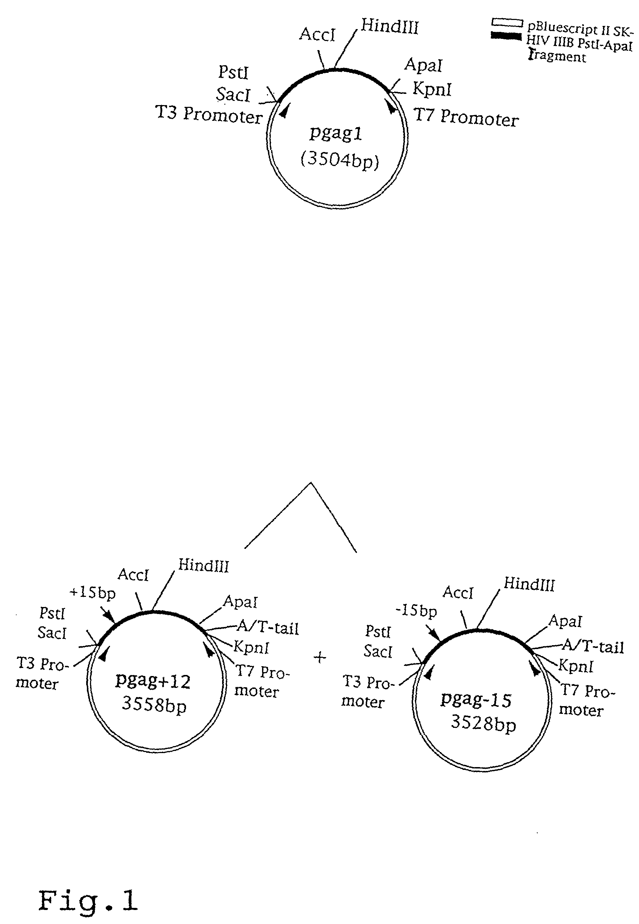

In pgag-15 wurden die bp 1593 bis 1607 deletiert, in pgag+12 ein 12 bp langes Insert an der Stelle 1593 eingefügt (siehe Fig.1). Die Plasmide wurden gereinigt (QUIAGEN-Verfahren), die Konzentration durch spektroskopische Messung bei 260 nm bestimmt und in einem 10mM TRIS/HCI pH 8/0,1 mM EDTA-Puffer verdünnt (Sambrook et al. Molecular Cloning, Second Edition, Cold Spring Harbor Lab Press, Cold Spring Harbor (1989)).

Nach Linearisierung mit Asp718 ergibt die in vitro Transkription mit T3-Polymerase gemäß Sambrook et al. ein 644 "+"-Transkript und ein 617 b "-"-Transkript, welche mit einer Guanidinisothiocyanatlösung extrahiert und durch spektrophotometrische Messungen bei 260 nm quantifiziert werden.

Diese RNA-Präparationen dienen als Standard für die RT-PCR.

Die Länge der RT-PCR-Produkte von Standard und Wildtyp-DNA betragen daher 127 (pgag+12), 100 (pgag-15) und 115 bp (wt).

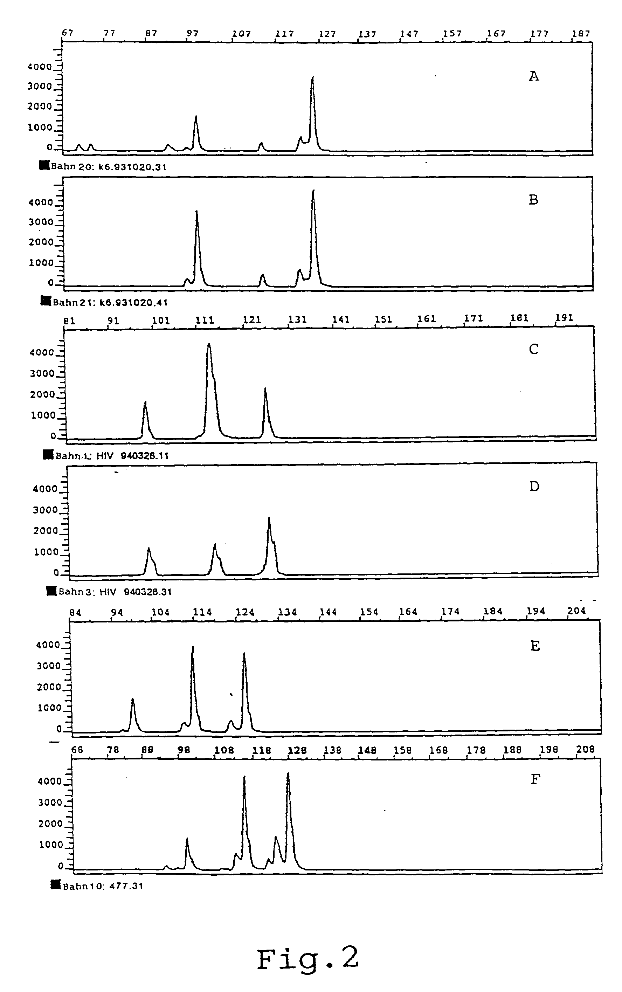

Die Figuren 2A und 2B illustrieren die Ergebnisse eines Kontrollexperiments. Zwei Proben mit je 100 Kopien wt-RNA, hergestellt von dem Plasmid pgag1, nach oben beschriebener Methode extrahiert, durch Koamplifikation mit "-"- und "+"-Standard amplifiziert und durch "Genescanning" analysiert. In beiden Spuren sind die PCR-Produkte von "-"-, "wt"- und "+"-Standard zu erkennen. Die Resul-tate der quantitativen Auswertung dieser Chromatogramme sind in Tabelle 1, Zeilen 1 und 2, angegeben.

Die Spalten "N-added" und "N+added" geben die Anzahl von zugegebenen Kopien von "-"- und "+"-Standard an. "Dilution" gibt den Verdünnungsfaktor an, um den die Probe verdünnt wurde, "Volume" das aufgearbeitete Volumen. "Sample" gibt den Probencode an, "Comment" enthält weitere Informationen zur Probe. "GS#, Lane" gibt den Analysenlauf bzw. die Nummer der Spur in diesem Lauf an. "A-", "Awt" und "A+" geben die Peakflächen, also die Intensität der PCR-Pro-dukte von "-", "wt" und "+". "N-Base" bzw. "N+Base" enthält das Resultat, angegeben in Kopien pro ml Probe, berechnet nach oben angegebener Formel. Die Spalte "Final Result" gibt den Mittelwert dieser Werte an.

Für die Proben wurden 114 bzw. 106 Kopien gemessen, wobei beiden Proben 100 Kopien zugegeben wurde. Es ist festzustellen, daß zwischen theoretischem Wert (i.e. Kopien wt zugegeben) und gemessenem Wert (i.e. Kopien wt bestimmt) eine gute Übereinstimmung herrscht. Die Abweichungen betragen +14 bzw. +6 %. Daraus ist zu schließen, daß mit dieser Methode eine unbekannte Menge wt-RNA bestimmt werden kann.

Für ein weiteres Kontrollexperiment wurde ein negatives Plasma mit einer Präparation von HIVIIIB versetzt, deren Infektiosität zuvor in vitro bestimmt wurde. Die Viruskonzentration im Plasma betrug 200 $TCID_{50}$ pro ml. Je 0,5 ml einer 1:10 bzw. einer 1:40 Verdünnung wurden wie oben beschrieben analysiert. Die Ergebnisse sind in den Figuren 2C und 2D sowie in den Zeilen 3 und 4 der Tabelle 1 wiedergegeben. Für beide Proben ergibt sich eine Kopienzahl im unverdünnten Plasma von 33187 bzw. 25895 pro ml. Die Abweichung vom Mittelwert beträgt 12 %. Dieses Experiment zeigt, daß die gemessene Kopienzahl HIV in einer Probe unabhängig von der im Test eingesetzten Menge ist.

Die Figuren 2E und 2F sowie die Zeilen 5 und 6 der Tabelle 1 zeigen die Ergebnisse der Bestimmung einer unbekannten Probe. In zwei unabhängigen Bestimmungen wurden 5612 bzw. 5828 Kopien gemessen. Die Abweichung beträgt 2 %. Daraus ist zu schließen, daß die oben beschriebene Methode zur sensitiven und präzisen Bestimmung von HIV geeignet ist.

Die höhere Abweichung bei Messung von verschiedenen Verdünnungen der gleichen Probe kann dem zusätzlichen Fehler durch das Verdünnen zugeschrieben werden.

## TABELLE 1

| | N-zugeg | N-zugeg | Verdünnung | Vol. | Probe | Erläuterung | N-Base | N+Base | Endergebnis | GS#Bahn | A- | Awt | A+ |
|---|---|---|---|---|---|---|---|---|---|---|---|---|---|
| 1 | 500 | 1500 | 1 | 1 | k6.931020.31 | 100 cop. wt zug. | 109 | 120 | 114 | 126,20 | 10303 | 2254 | 28092 |
| 2 | 500 | 1500 | 1 | 1 | k6.931020.41 | 100 cop. wt zug. | 76 | 136 | 106 | 126,21 | 22353 | 3441 | 37739 |
| 3 | 250 | 750 | 10 | 0.5 | HIV 940328.11 | Kontrollplasma | 21079 | 45296 | 33187 | 194,01 | 11199 | 47214 | 15635 |
| 4 | 250 | 750 | 40 | 0.5 | HIV 940328.31 | Kontrollplasma | 22821 | 20969 | 25895 | 194,03 | 10400 | 11867 | 24578 |
| 5 | 500 | 1500 | 1 | 0.25 | 477.21 | Probe | 5349 | 5875 | 5612 | 147,01 | 12181 | 32580 | 33270 |
| 6 | 500 | 1500 | 1 | 0.25 | 477.31 | Probe | 7460 | 4197 | 5828 | 149,10 | 10981 | 40964 | 58561 |

3. Beispiel 2: Quantifizierung von HAV durch RT-PCR

Bei dieser Quantifizierung werden Primer verwendet, welche in den cDNA-Sequenzen des HAV binden und durch RT-PCR von Wildtyp-RNA ein 139 bp großes Produkt ergeben, nämlich

```
HAV+2058:ACTGCCATTGGGAAGCTTATTGTG      HAV 2058-2081   Seq.ID 3
```

und

```
HAV-2172:CATCCATAGCATGATAAAGAGGAGC     HAV 2196-2172   Seq.ID 4
```

(Numerierung nach Cohen et al. (J.Virol.61 (1987) 50-59). Die Primer wurden unter Verwendung der Phosphoamidit-Chemie auf einem DNA-Synthesizer hergestellt (Applied Biosystems 394 DNA Synthesizer).

Die Standard-Plasmide pHAV-10 und pHAV+9 sind abgeleitet aus dem Plasmid pHAV-wt, welches aus dem bekannten pCRII-Plasmid (Firma InVitrogen) und einem Insert in der multiplen Klonierungsstelle dieses Plasmids besteht, welches Insert die bp 2020 bis 2226 des HAV aus Cohen et al. enthält.

In pHAV-10 wurden die bp 2100 bis 2109 deletiert, in pHAV+9 ein 9 bp langes Insert an der Stelle 2100 eingefügt. Die Plasmide wurden gereinigt (QUIAGEN-Verfahren), die Konzentration durch spektroskopische Messung bei 260 nm bestimmt und in einem 10mM TRIS/HCl pH 8/0,1 mM EDTA-Puffer verdünnt (Sambrook et al. Molecular Cloning, Second Edition, Cold Spring Harbor Lab Press, Cold Spring Harbor (1989)).

Nach Linearisierung mit AlwNI ergibt die in vitro Transkription mit T7-Polymerase gemäß Sambrook et al. ergibt ein 1140 b "+"-Transkript, ein 1121 b "-"-Transkript und ein 1131 b "wt"-Transkript, welche mit einer Guanidinisothiocyanatlösung extrahiert und durch spektrophotometrische Messungen bei 260 nm quantifiziert werden.

Diese RNA-Präparationen dienen als Standard für die RT-PCR.

Die Länge der RT-PCR-Produkte von Standard und Wildtyp-DNA betragen 148 (pHAV+9), 129 (pHAV-10) und 139 bp (wt).

Zwei Plasmen (PL1 und PL2) sowie zwei Albuminlösungen (Albumin und Humanalbumin) wurden mittels der beschriebenen Methode auf HAV untersucht. Tabelle 2 zeigt die Auswertung der Messungen mit dem Nukleinsäure-Detektionsgerät mit Hilfe eines Computerprogrammes (MS Excel® ). Spalten 1 und 2 geben die Menge an eingesetzten Minus-Standard und Plus-Standard an. Spalten 3 und 4 bezeichnen Verdünnung und eingesetztes Volumen. In Spalte 5 ist die Probe angegeben, Spalte 6 bezeichnet das Virus, auf das untersucht wird. Die Kopienzahlen der Proben wurden sowohl anhand des Minus-Standards (N-Base; Spalte 7) als auch anhand des Plus-Standards (N+Base; Spalte 8) berechnet; der Mittelwert beider Bestimmungen ergibt das Meßergebnis. Spalte 9 blieb leer. Spalte 10 gibt die Nummer des Probenlaufes an. Die Spalten 11, 12 und 13 geben die Fläche der detektierten Peaks an.

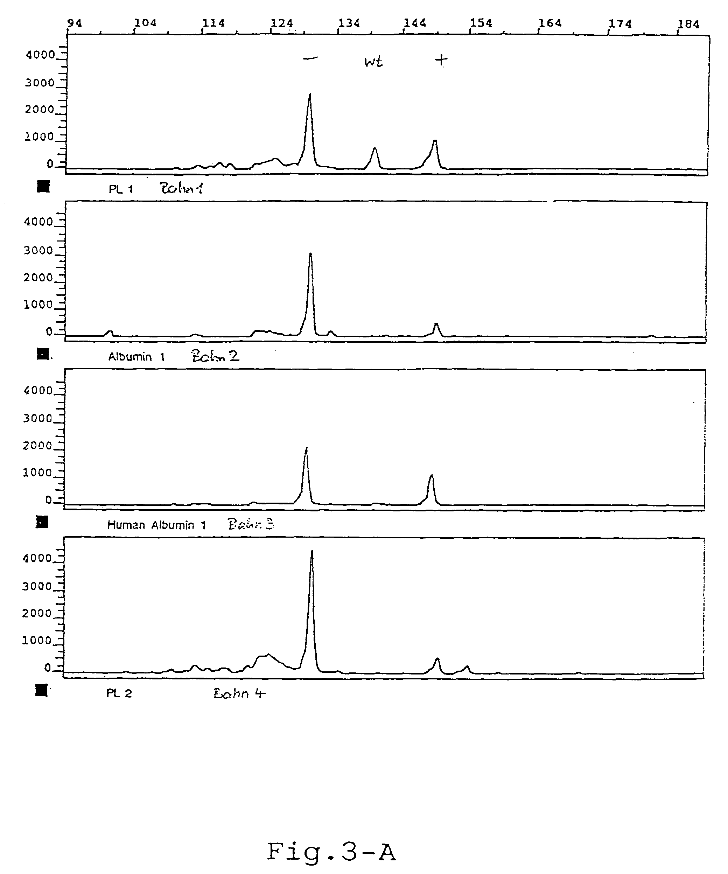

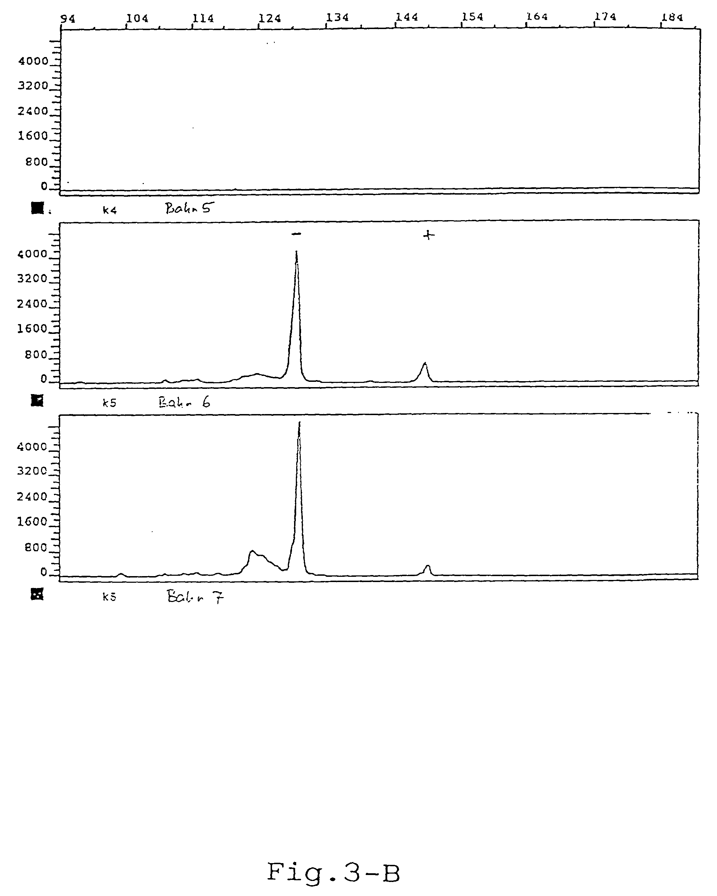

Fig.3 zeigt die graphische Auswertung der HAV-Untersuchung, wobei in den verschiedenen Bahnen die Intensitäten der Fluoreszenzsignale der PCR-Produkte (und Nebenprodukte) dargestellt. Die Produkte sind anhand ihrer definierten Größe (in bp) identifizierbar. Die Standards sind 148 und 129 bp lang, der Wildtyp 139.

TABELLE 2

| 1 | 2 | 3 | 4 | 5 | 6 | 7 | 8 | 9 | 10 | 11 | 12 | 13 |
|---|---|---|---|---|---|---|---|---|----|----|----|----|
| 900 | 300 | 1 | 0,25 | PL 1 | HAV | 910 | 677 | - | 314, 17 | 22213 | 5066 | 10040 |
| 900 | 300 | 1 | 0,25 | Albumin 1 | HAV | -1 | -1 | - | 314, 18 | 19005 | -1 | 2814 |
| 900 | 300 | 1 | 0,25 | Human Albumin 1 | HAV | -1 | -1 | - | 314, 19 | 13995 | -1 | 7308 |
| 900 | 300 | 1 | 0,25 | PL 2 | HAV | -1 | -1 | - | 314, 20 | 29203 | -1 | 4278 |

Die Ergebnisse dieser Untersuchungen zeigten, daß Plasma 1 positiv war (798 Kopien/ml), während die anderen Proben unterhalb der Nachweisgrenze lagen. Der "Nachweisgrenzenpeak" (der Plus-Standard in einer Kopienzahl von 300 Kopien/ml extrahiertes Material) ist in allen Messungen klar erkennbar.

4. Beispiel 3: Quantifizierung von HCV durch RT-PCR

Bei dieser Quantifizierung werden Primer verwendet, welche in den cDNA-Sequenzen des HCV binden und durch RT-PCR von Wildtyp-RNA ein 114 bp großes Produkt ergeben, nämlich

```
HCV32EXT:    CTGTGAGGAACTACTGTCTTACGCAG    HCV 45-70   Seq.ID 5 und
HCVPT4:      CGGTTCCGCAGACCACTATG          HCV 158-139    Seq ID 6
```

(Numerierung nach Han et al. (PNAS 88 (1991) 1711-1715). Die Primer wurden unter Verwendung der Phosphoamidit-Chemie auf einem DNA-Synthesizer hergestellt (Applied Biosystems 394 DNA Synthesizer).
Die Standard-Plasmide pHCV-7 und pHCV+8 sind abgeleitet aus dem Plasmid pHCV-wt, welches aus dem bekannten pBS/SK⁻-Plasmid (Firma Statagene) und einem Insert in der multiplen Klonierungsstelle dieses Plasmids besteht, welches Insert die bp 27 bis 313 des HCV aus Han et al. enthält.
In pHCV-7 wurden die bp 126 bis 135 deletiert, in pHCV+8 ein 8 bp langes Insert an der Stelle 126 eingefügt. Die Plasmide wurde gereinigt (QUIAGEN-Verfahren), die Konzentration durch spektroskopische Messung bei 260 nm bestimmt, mit XmnI geschnitten und in einem 10mM TRIS/HCl pH 8/0,1 mM EDTA-Puffer verdünnt (Sambrook et al., Molecular Cloning, Second Edition, Cold Spring Harbor Lab Press, Cold Spring Harbor (1989)).
In vitro-Transkription mit T3-Polymerase gemäß Sambrook et al. ergibt ein 1385 b "+"Transkript, ein 1370 b "-"Transkript und ein 1377 b "wt"Transkript, welche mit einer Guanidinisothiocyanatlösung extrahiert und durch spektrophotometrische Messungen bei 260 nm quantifiziert werden.
Diese RNA-Präparationen dienen als Standard für die RT-PCR.
Die Länge der RT-PCR-Produkte von Standard und Wildtyp-DNA betragen daher 122 (pHCV+8), 107 (pHCV-7) und 114 bp (wt).
Zwei Proben mit 200bzw. 2000 Kopien wt-RNA, hergestellt von dem Plasmid pHCV-wt, wurden nach oben beschriebener Methode extrahiert, durch Koamplifikation mit "-"- und "+"-Standard amplifiziert und durch "Genescanning" analysiert. In beiden Spuren sind die PCR-Produkte von "-"-, "wt"- und "+"-Standard zu erkennen. Die Resultate der quantitativen Auswertung dieser Chromatogramme sind in den Zeilen 1 und 2 der Tabelle 3 angegeben. Die Tabelle ist analog zu Beispiel 1 aufgebaut.
Für die Proben wurden 1972 bzw. 193 Kopien gemessen, wobei den Proben 2000 bzw. 200 Kopien zugegeben wurden. Es ist festzustellen, daß zwischen theoretischem Wert (i.e. Kopien wt zugegeben) und gemessenem Wert (i. e. Kopien wt bestimmt) eine gute Übereinstimmung herrscht. Die Abweichungen betragen -1 bzw. -3 %. Daraus ist zu schließen, daß mit dieser Methode eine unbekannte Menge wt-RNA bestimmt werden kann.
Für ein weiteres Kontrollexperiment wurde ein Pool von HCV-positivem Plasma von verschiedenen Patienten verwendet. Je 0,5 ml einer 1:125 bzw. einer 1:625-Verdünnung wurden wie oben beschrieben analysiert. Die Ergebnisse sind in den Zeilen 3 und 4 der Tabelle 3 wiedergegeben. Für beide Proben ergibt sich eine Kopienzahl im unverdünnten Pool von 1215157 bzw. 898327 pro ml. Die Abweichung vom Mittelwert beträgt 15 %. Dieses Experiment zeigt, daß die gemessene Kopienzahl HCV in einer Probe unabhängig von der im Test eingesetzten Menge ist. Dieses Beispiel zeigt ferner, daß die Reproduzierbarkeit des erfindungsgemäßen Verfahrens nicht nur im Bereich geringer Kopienzahl, sondern auch im Bereich hoher Kopienzahl gegeben ist.
Die Zeilen 5 und 6 der Tabelle 3 zeigen die Ergebnisse der Bestimmung einer unbekannten Probe. In zwei unabhängigen Bestimmungen wurden 3277 bzw. 3676 Kopien gemessen. Die Abweichung beträgt 5 %. Daraus ist zu schließen, daß die oben beschriebene Methode zur sensitiven und präzisen Bestimmung von HCV geeignet ist.
Die höhere Abweichung bei Messung von verschiedenen Verdünnungen der gleichen Probe kann dem zusätzlichen Fehler durch das Verdünnen zugeschrieben werden.

TABELLE 3

| | N- zugeg. | N+ zugeg. | Verdünnung | Vol. | Probe | Erläuterung | N -Base | N+Base | Endergebnis | GS/Bahn. | A- | A wt | A + |
|---|---|---|---|---|---|---|---|---|---|---|---|---|---|
| 1 | 400 | 1200 | 1 | 1 | HCV 940314 21 | 2000 Kopien wt | 2915 | 1030 | 1972 | 181. 02 | 1053 | 7676 | 8942 |
| 2 | 400 | 1200 | 1 | 1 | HCV 940314 31 | 200 Kopien wt | 234 | 152 | 193 | 181. 03 | 1100 | 844 | 5052 |
| 3 | 400 | 1200 | 125 | 0.5 | HCV 940328 41 | Plasma | 1864107 | 566208 | 1215157 | 192. 12 | 560 | 10439 | 5551 |
| 4 | 400 | 1200 | 625 | 0.5 | HCV 940328.51 | Plasma | 637312 | 1159343 | 898327 | 192. 13 | 4264 | 5435 | 7032 |
| 5 | 400 | 1200 | 1 | 1 | Probe 1A | keine | 4563 | 1992 | 3277 | 204. 03 | 492 | 5613 | 3380 |
| 6 | 400 | 1200 | 1 | 1 | Probe 1B | keine | 3690 | 3662 | 3676 | 204. 04 | 614 | 5665 | 1856 |

17

5. Beispiel 4: Quantifizierung von HIV-proviraler DNA

Bei dieser Quantifizierung werden Primer verwendet, welche in den cDNA-Sequenzen des HIV-1 binden und durch PCR von proviraler HIV- DNA ein 115 bp großes Produkt ergeben, nämlich

```
SK38:   ATAATCCACCTATCCCAGTAGGAGAAAT HIV-1 1551-1578   Seq.ID 1
SK39:   TTTGGTCCTTGTCTTATGTCCAGAATGC HIV-1 1665-1638   Seq.ID 2
```

(Numerierung nach Ratner et al.). Die Primer wurden unter Verwendung der Phosphoamidit-Chemie auf einem DNA-Synthesizer hergestellt (Applied Biosystems 394 DNA Synthesizer).

Die Standard-Plasmide pgag-15 und pgag+12 sind abgeleitet aus dem Plasmid pgag1, welches aus dem bekannten pBS/SK⁻-Plasmid (Firma Stratagene) und einem Insert in der multiplen Klonierungsstelle dieses Plasmids besteht, welches Insert die bp 1417 bis 2008 der HIV-1 aus Ratner et al. enthält.

In pgag-15 wurden die bp 1593 bis 1607 deletiert, in pgag+12 ein 12 bp langes Insert an der Stelle 1593 eingefügt (siehe Fig.1). Die Plasmide wurde gereinigt (QUIAGEN-Verfahren), die Konzentration durch spektroskopische Messung bei 260 nm bestimmt, mit EcoRI geschnitten und in einem 10mM TRIS/HCl pH 8/0,1 mM EDTA-Puffer verdünnt (Sambrook et al. Molecular Cloning, Second Edition, Cold Spring Harbor Lab Press, Cold Spring Harbor (1989)).

Diese DNA-Präparationen dienen als Standard für die PCR.

Die Länge der PCR-Produkte von Standard und Wildtyp-DNA betragen daher 127 (pgag+12), 100 (pgag-15) und 115 bp (wt).

Die Ergebnisse einer Quantifizierungsreihe sind in Tabelle 4 wiedergegeben. Eine kodierte Kontrollreihe von positiven und negativen Proben, die HIV provirale DNA in unterschiedlicher Kopienzahl enthielten, wurden mit dem erfindungsgemäßen Verfahren bestimmt. Die Tabelle 4 zeigt, daß alle Negativkontrollen negativ und alle Positiv-Proben im richtigen Größenbereich quantifiziert wurden. Besonders bemerkenswert ist die genaue Messung im unteren Kopienbereich, siehe CD-13 und CD-26. Diese Proben enthielten nominal 2 Kopien/$10^5$-Zellen und wurden mit jeweils 3 Kopien quantifiziert. Dies zeigt die Genauigkeit und extreme Sensitivität des erfindungsgemäßen Verfahren.

TABELLE 4

| PROBE | KOPIENZAHL | | PROBE | KOPIENZAHL | |
|-------|------|------|-------|------|------|
|       | nom. | gef. |       | nom. | gef. |
| CD-1  | 0    | 0    | CD-16 | 0    | 0    |
| CD-2  | 50   | 68   | CD-17 | 0    | 0    |
| CD-3  | 0    | 0    | CD-18 | 0    | 0    |
| CD-4  | 0    | 0    | CD-19 | 2    | 5    |
| CD-5  | 10   | 24   | CD-20 | 20   | 16   |
| CD-6  | 0    | 0    | CD-21 | 0    | 0    |
| CD-7  | 0    | 0    | CD-22 | 0    | 0    |
| CD-8  | 10   | 16   | CD-23 | 10   | 18   |
| CD-9  | 20   | 49   | CD-24 | 0    | 0    |
| CD-10 | 0    | 0    | CD-25 | 0    | 0    |
| CD-11 | 5    | 6    | CD-26 | 2    | 3    |
| CD-12 | 5    | 5    | CD-25 | 50   | 71   |
| CD-13 | 2    | 3    | CD-28 | 0    | 0    |
| CD-14 | 0    | 0    | CD-29 | 5    | 5    |
| CD-15 | 0    | 0    | CD-30 | 20   | 25   |

6. Beispiel 5: Quantifizierung von HBV

Bei dieser Quantifizierung werden Primer verwendet, welche im Genom des HBV binden und durch PCR von Wildtyp-DNA ein 182 bp großes Produkt ergeben, nämlich

```
HBV+1780B:CATTGATCCTTATAAAGAATTTGGAGC HBV 1780-1806  Seq.ID 7
```

und

```
HBV-1960B:CCAGCAGAGAATTGCTTGCCTGAG    HBV 1983-1960  Seq.ID 8
```

(Numerierung nach Fujiyama et al.). Die Primer wurden unter Verwendung der Phosphoamidit-Chemie auf einem DNA-Synthesizer hergestellt (Applied Biosystems 394 DNA Synthesizer).

Die Standard-Plasmide pHBV-9 und pHBV+12 sind abgeleitet aus dem Plasmid pHBV-wt, welches aus dem bekannten pCRII-Plasmid (Firma In Vitrogene) und einem Insert in der multiplen Klonierungsstelle dieses Plasmids besteht, welches Insert die bp 1763 bis 1868 der HBV aus Fujiyama et al. enthält.

In pHBV-9 wurden die bp 1868 bis 1876 deletiert, in pHBV+12 ein 12 bp langes Insert an der Stelle 1868 eingefügt. Die Plasmide wurden gereinigt (QUIAGEN-Verfahren), die Konzentration durch spektroskopische Messung bei 260 nm bestimmt, mit einem Restriktionsenzym einmal geschnitten und in einem 10mM TRIS/HCl pH 8/0,1 mM EDTA-Puffer verdünnt (Sambrook et al. Molecular Cloning, Second Edition, Cold Spring Harbor Lab Press, Cold Spring Harbor (1989)).

Diese DNA-Präparationen dienen als Standard für die PCR.

Die Länge der PCR-Produkte von Standard und Wildtyp-DNA betragen daher 194 (pHBV+12), 173 (pHBV-9) und 182 bp (wt).

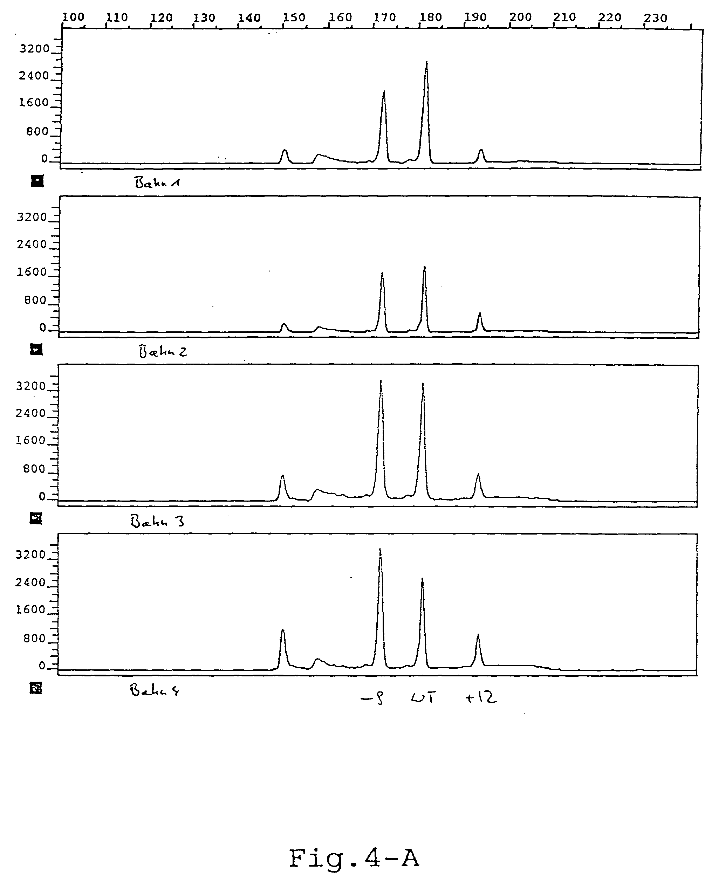

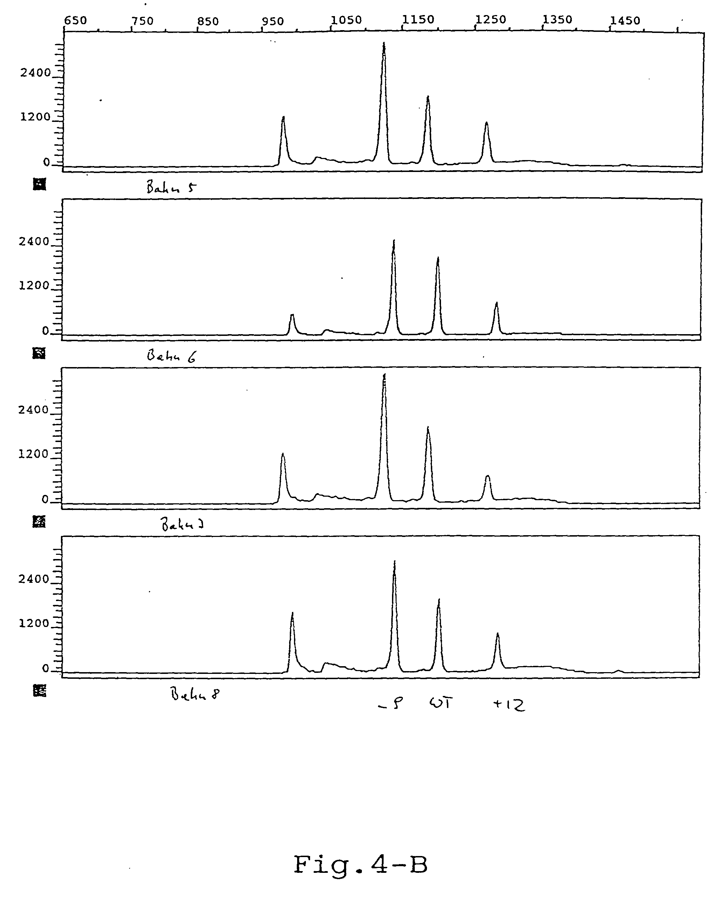

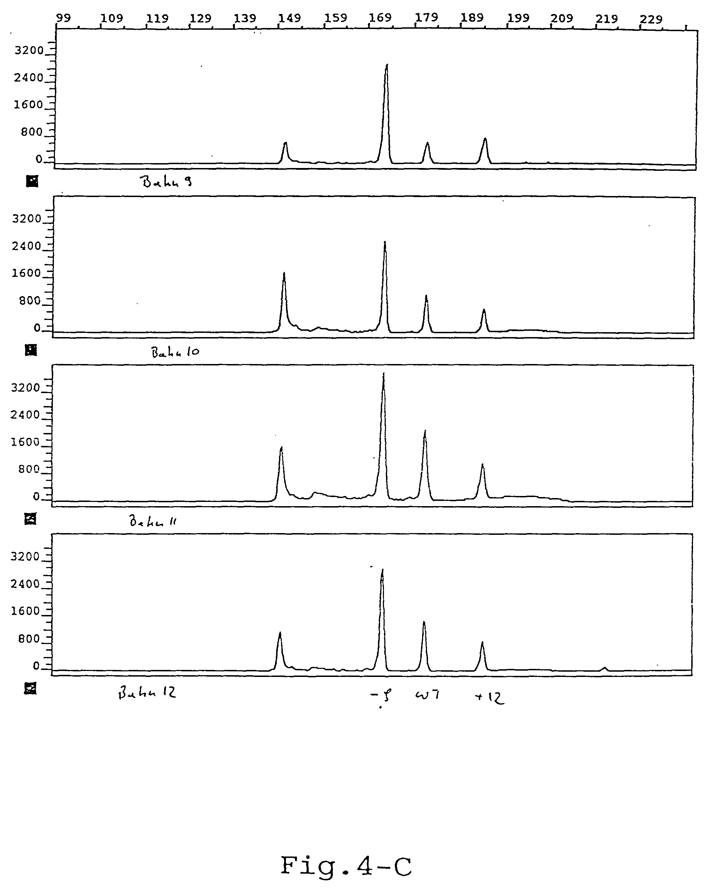

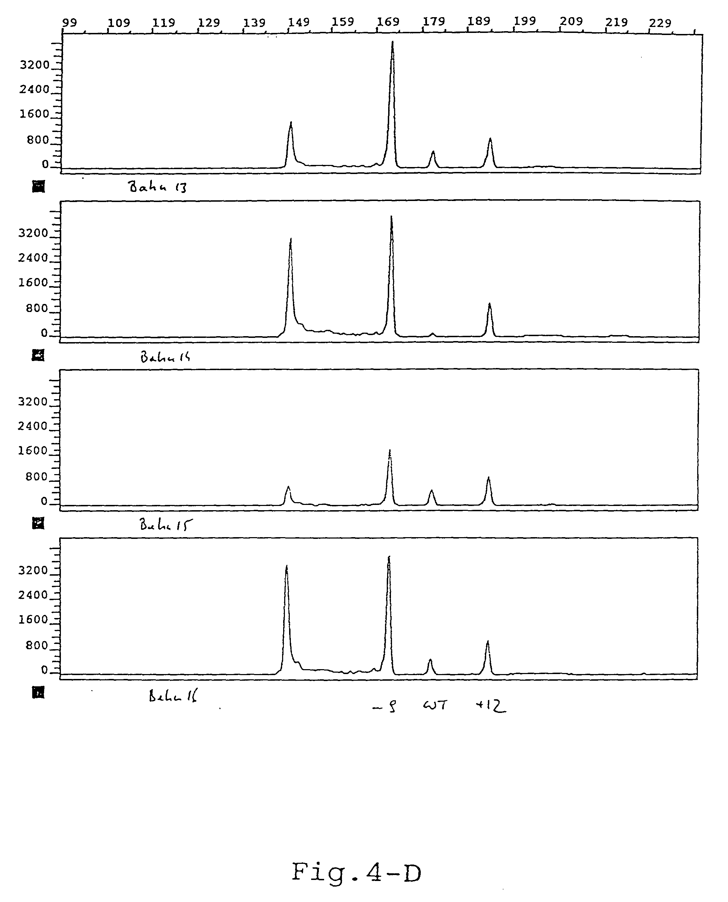

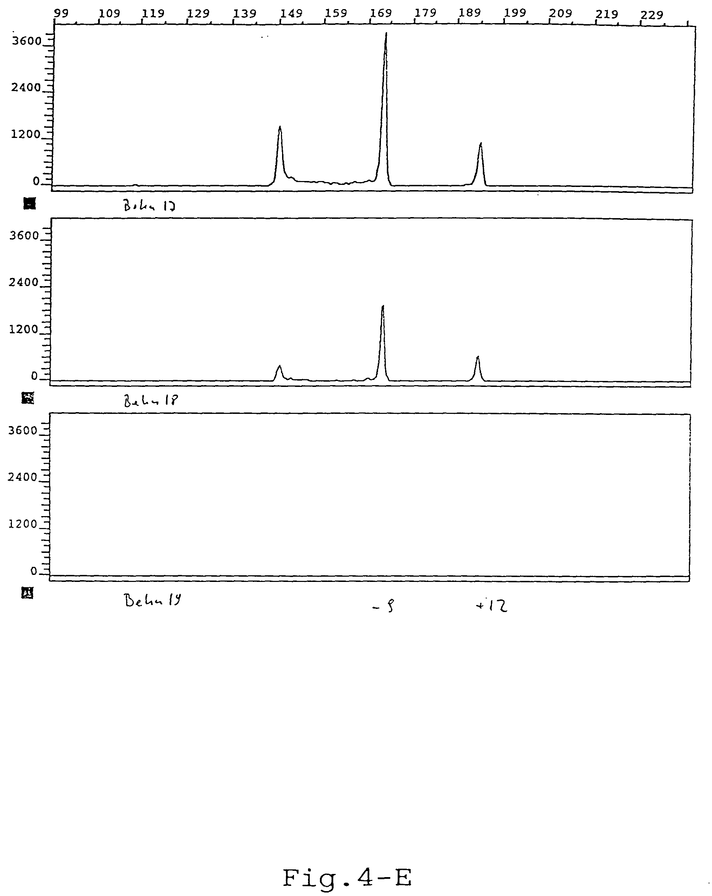

Die Ergebnisse einer Quantifizierungsreihe sind in Tabelle 5 wiedergegeben und in der Figur 4 graphisch veranschaulicht. Die Amplifizierungsreaktion wurde ausgehend von jeweils 150 Kopien pHBV-9 und 50 Kopien von HBV+12 und unterschiedlichen Mengen an pHBV-wt (400, 200, 100, 50 und 0 Kopien) durchgeführt. Jeder Ansatz wurde vierfach gemessen.

## TABELLE 5

| BAHN | CODE | A-9 | A-WT | A+12 | Kopien-9 | Kopien+12 | KOPIEN 1 | KOPIEN 2 | DURCHSCHNITTERGEBNISSE | A+12/A-15 |
|------|------|------|------|------|----------|-----------|----------|----------|------------------------|-----------|
| Bahn 1 | 400Kp. | 11405 | 13730 | 4461 | 150 | 50 | 301,2 | 307,8 | 334,5 | 2,6 |
| Bahn 2 | 400Kp. | 33124 | 31652 | 9660 | 150 | 50 | 296,7 | 327,4 | 307,0 | 3,4 |
| Bahn 3 | 200Kp. | 18698 | 15523 | 6960 | 150 | 50 | 250,4 | 223,0 | 236,7 | 2,7 |
| Bahn 4 | 200Kp. | 30630 | 19323 | 8380 | 150 | 50 | 189,3 | 230,8 | 209,9 | 3,7 |
| Bahn 5 | 100Kp. | 24400 | 5270 | 6731 | 150 | 50 | 64,8 | 78,3 | 71,6 | 3,6 |
| Bahn 6 | 100Kp. | 18788 | 7532 | 5581 | 150 | 50 | 102,4 | 135,0 | 127,7 | 3,4 |
| Bahn 7 | 50Kp. | 34103 | 4244 | 6193 | 150 | 50 | 37,3 | 51,8 | 44,8 | 5,5 |
| Bahn 8 | 50Kp. | 20818 | 3591 | 8570 | 150 | 50 | 37,4 | 41,8 | 39,8 | 2,4 |
| Bahn 9 | K6 | 33264 | -1 | 10199 | 150 | 50 | 0,0 | 0,0 | 0,0 | |
| Bahn 10 | K6 | 15217 | -1 | 5303 | 150 | 50 | 0,0 | 0,0 | 0,0 | |
| Bahn 11 | K3 | -1 | -1 | -1 | | | | | | |
| Bahn 12 | K3 | -1 | -1 | -1 | | | | | | |

Die Ergebnisse zeigen, daß der festgestellte DNA-Gehalt sehr gut reproduzierbar ist.

7. Beispiel 6: Quantifizierung der DNA von Herpes Simples-Virus (HSV)

Bei dieser Quantifizierung wurden Primer verwendet, welche im Genom des HSV binden und durch PCR von Wildtyp-DNA ein 114 bp langes Produkt ergeben, nämlich

```
gDR1/B:      AAC TAC CCC GAT CAT CAG          Seq.ID 11

GDR2R/B:     AGG CCC ACT TAG ACG ACA          Seq.ID 12
```

Das Standard-Plasmid pHerp-9 ist abgeleitet aus dem Plasmid pHerp, welches aus dem bekannten pCRII-Plasmis (Firma In Vitrogen) und einem Insert von bp128364 bis 138784 des HSV-Genoms (McGeoch D.J. et al., EMBL GenBank ID HEICG1) besteht.

In pHerp-9 wurden 9bp deletiert. Die Plasmide wurden gereinigt, die Konzentrationen durch spektroskopische Messung bei 260nm bestimmt, mit einem Restriktionsenzym linearisiert und in einem TE-Puffer (10 mM Tris/HCl, 1mM EDTA, pH 8) verdünnt.

Dieses DNA-Präparat dient als Standard für die PCR. Die Länge der PCR-Produkte von Standard und Wildtyp betragen daher 105 (pHerp-9) und 114 bp (pHerp).

Die Ergebnisse einer Quantifizierung sind in der Tabelle 6 wiedergegeben. Fünf Proben sowie 1,66 pg pHerp Wildtyp DNA wurden in Anwesenheit von 40000 (Spalte 1) Kopien Standard pHerp-9 mittels der beschriebenen Methode analysiert. Der Faktor F2 der beschriebenen Formel zur Berechnung der Herpes DNA-Menge ist in diesem Fall 1/6000. Die Spalten 2 und 3 geben den Verdünnungsfaktor sowie das eingesetzte Volumen der Probe an. Spalte 4 gibt den Namen der Probe an. Spalte 5 enthält zusätzliche Informationen über die Proben (wenn nötig). In der Spalte 6 ist die errechnete Menge an HSV-DNA in pg/ml angegeben. Der Mittelwert der Doppelbestimmung ist in Spalte 7 angegeben. Spalte 8 und 9 geben die Flächen der detektierten Peaks von Standard-Plasmid (Spalte 8) und von Wildtyp (Spalte 9) an.

Bei den Proben 14, 17 und JA10 werden Doppelbestimmungen von Herpes-DNA gemacht, die eine Abweichung vom Mittelwert von 0 % bis 4,3 % aufweisen. Diese geringe Standardabweichung kann noch dra-stisch verbessert werden, wenn man bei den Bestimmungen auch den pHerp+10-Standard mitbestimmt.

## TABELLE 6

| | N-zugegeben | Verdünnung | Vol. | Probe | Erläuterung | pg/ml | Endergebnis | A- | Awt |
|---|---|---|---|---|---|---|---|---|---|
| | 40000 | 1 | 0,5 | 0000.00 | Herpes | 1333. | | 100 | 10000 |
| | 40000 | 1 | 0,5 | Probe 14.11 | . | 3,1 | 3 | 7872 | 1840 |
| | 40000 | 1 | 0,5 | Probe 14.12 | . | 2,9. | | 8171 | 1774 |
| | 40000 | 1 | 0,5 | Probe 16.11 | . | 5,5 | 6,75 | 11105 | 4626 |
| | 40000 | 1 | 0,5 | Probe 16.12 | . | 6 | | 7286 | 3311 |
| | 40000 | 1 | 0,5 | Probe 17.11 | . | 1,7 | 1,7 | 12402 | 1570 |
| | 40000 | 1 | 0,5 | Probe 17.12 | . | 1,7. | | 9118 | 1181 |
| | 40000 | 1 | 0,5 | JA 10.11 | . | 1,8 | 1,65 | 10713 | 1316 |
| | 40000 | 1 | 0,5 | JA 10.12 | . | 1,7. | | 9200 | 1148 |
| | 40000 | 1 | 0,5 | H2O | . | 0 | 0 | 7843 | -1 |
| | 40000 | 1 | 0,5 | H2O | . | 0 | | 4077 | -1 |
| | 40000 | 1 | 0,5 | PCR | . | 0 | 0 | -1 | -1 |
| | 40000 | 1 | 0,5 | PCR | . | 0 | | -1 | -1 |
| | 10000 | 1 | 1 | 1,00pg wt 1.2 | . | 1,8. | | 3863 | 4352 |
| | 1 | 2 | 3 | 4 | 5 | 6 | 7 | 8 | 9 |

8. Beispiel 7: Quantifizierung von Vaccinia-DNA

Bei dieser Quantifizierung wurden Primer verwendet, welche im Genom des Vaccinia-Virus binden und durch PCR von Wildtyp-DNA ein 136 bp langes Produkt ergeben, nämlich

```
oprl-r      AAA ATA GGA TCA TGA TGG C      bp 84626-84644
oprl-f      ATA TTA GAT GGT GCC ACC GT     bp 84761-84743
```

(Numerierung nach Goebel et al., Virology 179: 247-266, 1990). Die Primer wurden auf einem DNA-Synthesizer hergestellt (Applied Biosystems 394 DNA Synthesizer).

Das Standard-Plasmid pVV-21 ist abgeleitet aus dem Plasmid pVVwt, welches aus dem bekannten pTZ19R-Plasmid (Pharmacia) und einem Insert zwischen den beiden PvuII-Restriktionsssschnittstellen dieses Plasmids besteht. Dieses Insert enthält die bp 83855 bis 84761 der Thymidinkinasegen-Region des Vaccinia-Virus (siehe Goebel et al., 1990).

In pVV-21 wurden 21 bp (von bp 84718 bis 84738) deletiert.

Die Plasmide wurden gereinigt, die Konzentrationen durch spektroskopische Messung bei 260 nm bestimmt, mit einem Restriktionsenzym linearisiert und in einem TE-Puffer (10 mM Tris/HCl, 1mM EDTA, pH 8) verdünnt.

Dieses DNA-Präparat dient als Standard für die PCR. Die Länge der PCR-Produkte von Standard und Wildtyp betragen daher 115 (pVV-21) und 136 (pVVwt).

Die Ergebnisse einer Quantifizierung sind in der Tabelle 7 wiedergegeben. Proben, Wasser und 10 pg Vaccinia-DNA wurden in Anwesenheit von 10000 Kopien Standard-Plasma (Spalte 1) mittels der beschriebenen Methode analysiert. Der Wert des Faktors F2 der beschriebenen Formel für die Berechnung der Vaccinia-DNA in pg/ml ist in diesem Fall 1/5000.

Die Spalten 2 und 3 geben den Verdünnungsfaktor sowie das eingesetzte Volumen der Probe an. In Spalte 4 können (wenn nötig) mehr Informationen über die Probe angegeben werden. Spalte 5 gibt den Namen der Probe an. In Spalte 6 ist die berechnete Menge an Vaccinia-DNA in pg/ml angegeben. Spalte 7 gibt die Nummer des Genescanner-Laufes an, in dem die PCR-Produkte analysiert worden sind. Spalte 8 und 9 geben die Flächen der detektierten Peaks von Standard-Plasmid (Spalte 8) und von Wildtyp (Spalte 9) an.

Bei der Probe 311494 werden 1,64 pg, bzw. 1,77 pg Vaccinia-DNA gemessen, wobei die Abweichung vom Mittelwert bei 3,8 % liegt. Bei der Probe 310494 werden 7,0 pg, bzw. 7,2 pg Vaccinia-DNA gemessen, wobei die Abweichung vom Mittelwert bei 1,4 % liegt. Diese geringe Standardabweichung kann noch drastisch verbessert werden, wenn man bei den Bestimmungen auch den pVV+24-Standard mitbestimmt.

## TABELLE 7

| N-zugegeben | Verdünnung | Volumen | Probe | Erläuterung | pg/ml | GS#, Bahn | A- | Awt |
|---|---|---|---|---|---|---|---|---|
| 45000 | 1 | 0,5 | 0000.00 | keine | 1800 | 261, 00 | 100 | 10000 |
| 40000 | 1 | 1 | 10pg | keine | 10,6 | 259, 06 | 35636 | 47165 |
| 40000 | 5 | 0,5 | 310494 | keine | 7 | 216, 09 | 55109 | 4820 |
| 40000 | 5 | 0,5 | 310494 | keine | 7,2 | 216, 01 | 37686 | 3375 |
| 40000 | 5 | 0,5 | 311494 | keine | 1,64 | 246, 18 | 49362 | 1013 |
| 40000 | 5 | 0,5 | 311494 | keine | 1,77 | 246, 20 | 48816 | 1079 |
| 40000 | 1 | 0,5 | H2O | keine | -1 | 246, 21 | 41041 | -1 |
| 40000 | 1 | 0,5 | H2O | keine | -1 | 246, 24 | 46458 | -1 |
| 40000 | 1 | 1 | PCR | keine | 0 | 246, 27 | -1 | -1 |
| 1 | 2 | 3 | 4 | 5 | 6 | 7 | 8 | 9 |

## Patentansprüche

**1.** Verfahren zur Quantifizierung von Nukleinsäuren in einer Probe unter Anwendung von Nukleinsäure-Amplifizierung, wobei der Probe vor dem Amplifizierungsschritt eine gegebene Menge eines bekannten Nukleinsäuremoleküls als interner Standard zugegeben wird, welches Standard-Nukleinsäuremolekül sich von der zu quantifizierenden Nukleinsäure zumindest in einem detektierbaren Merkmal unterscheidet, **dadurch gekennzeichnet, daß** der Probe vor der Nukleinsäure-Amplifizierung bekannte Mengen von mindestens zwei sich zumindest in einem detektierbaren Merkmal voneinander und von der zu quantifizierenden Nukleinsäure unterscheidenden bekannten Nukleinsäuremolekülen als interner Standard zugegeben werden, die erhaltenen Mengen an amplifizierter Proben- und Standard-Nukleinsäure bestimmt werden und aus den erhaltenen Mengen die ursprünglich in der Probe vorhandene Menge an zu quantifizierender Nukleinsäure bestimmt wird und daß beim Amplifizieren Primer mit fluoreszierenden oder radioaktiven Gruppen oder chemischen Gruppen, die mit affinen Proteinen und nachgeschalteten Detektionsreaktionen detektiert werden können, vorzugsweise mit fluoreszierenden Gruppen, verwendet werden.

2. Verfahren nach Anspruch 1, **dadurch gekennzeichnet, daß** die Amplifizierung der Nukleinsäuren bereits in der exponentiellen Phase gestoppt wird.

3. Verfahren nach Anspruch 1 oder 2, **dadurch gekennzeichnet, daß** die Bestimmung der Mengen an amplifizierter Nukleinsäure unter Verwendung eines Nukleinsäure-Detektionsgerätes, vorzugsweise eines Fluoreszenz-empfindlichen Nukleinsäure-Detektionsgerätes, erfolgt.

4. Verfahren nach einem der Ansprüche 1 bis 3, **dadurch gekennzeichnet, daß** Nukleinsäuren von Mikroorganismen, vorzugsweise HIV, Parvovirus, Herpesvirus, HAV, HBV, HCV, Baculovirus, Adenovirus, Vacciniavirus, Borreliaspezies, Salmonellaspezies und Hefe, quantifiziert werden.

5. Verfahren nach Anspruch 4, **dadurch gekennzeichnet, daß** virale Nukleinsäuren in einer biologischen Probe, insbesondere Blut- und Blutderivaten und biotechnologischen Produkten, quantifiziert werden.

6. Verfahren nach einem der Ansprüche 1 bis 5, **dadurch gekennzeichnet, daß** unterschiedliche Mengen der Standard-Nukleinsäuren der Probe vor der Amplifizierung zugegeben werden.

7. Verfahren nach einem der Ansprüche 1 bis 6, **dadurch gekennzeichnet, daß** Standard-Nukleinsäuren zum Einsatz kommen, welche eine gegenüber der zu quantifizierenden Nukleinsäure unterschiedliche Länge aufweisen, vorzugsweise eine Standard-Nukleinsäuresequenz, welche kürzer, und eine Standard-Nukleinsäuresequenz, welche länger ist als die zu quantifizierende Nukleinsäure.

8. Verfahren nach einem der Ansprüche 1 bis 7, **dadurch gekennzeichnet, daß** mehrere erhaltene Mengen an amplifizierter Proben- und Standard-Nukleinsäuren in der gleichen Probe mittels der Multiplex-Analyse bestimmt werden.

9. Verfahren nach einem der Ansprüche 1 bis 8, **dadurch gekennzeichnet, daß** die Standard-Nukleinsäuren der Probe zugegeben werden, noch bevor die Probe einem oder mehreren Probenaufbereitungsschritten unterzogen wird.

10. Verfahren zur Bestimmung der Nachweisgrenze von bestimmten Nukleinsäuren unter Verwendung eines Verfahrens gemäß der Ansprüche 1 bis 9, **dadurch gekennzeichnet, daß** mindestens einer Standard-Nukleinsäure mit einer Konzentration von knapp oberhalb der Nachweisgrenze eingesetzt wird.

11. Verwendung eines Verfahrens gemäß einem der Ansprüche 1 bis 9 zur Detektion von Nukleinsäuren in biologischen Proben.

12. Primer mit der Sequenz gemäß der Seq.ID 3, 4, 5, 7, 8, 9, 10, 11 und 12.

13. Verwendung eines Primers mit der Sequenz gemäß Seq.ID 6 in einem Verfahren gemäß einem der Ansprüche 1 bis 10.

14. pBS/SK-Plasmid mit einem Insert in der multiplen Klonierungsstelle, welches die bp1417-2008 des HIV I enthält [pgag-1] ; pBS/SK-Plasmid mit einem Insert in der multiplen Klonierungsstelle, welches die bp1417-2008 des HIV I enthält, wobei die bp1593-1607 deletiert sind [pgag-15] ; bpS/SK-Plasmid mit einem Insert in der multiplen Klonierungsstelle, welches die bp1417-2008 des HIV I enthält, wobei ein 12bp langes Insert an der Stelle 1593 eingefügt ist [pgag+12];

15. pCRII-Plasmid mit einem Insert in der multiplen Klonierungsstelle, welches die bp2020 bis 2226 der cDNA-HAV-Sequenz enthält [pHAV-wt]; pCRII-Plasmid mit einem Insert in der multipen Klonierungsstelle, welches die bp2020 bis 2226 der cDNA-HAV-Sequenz enthält, wobei die bp2100 bis 2109 der HAV-Sequenz deletiert sind [pHAV-10bp]; pCRII-Plasmid mit einem Insert in der multiplen Klonierungsstelle, welches die bp2020 bis 2226 der cDNA-HAV-Sequenz enthält, wobei ein 9 bp langes Insert an der Stelle 2100 eingefügt ist [pHAV+9bp];

16. pBS/SK-Plasmid mit einem Insert in der EcoRV-Stelle, welches die bp27 bis 313 der cDNA-HCV-Sequenz enthält [pHCV-wt]; pBS/SK-Plasmid mit einem Insert in der EcoRV-Stelle, welches die bp27 bis 313 der cDNA-HCV-Sequenz enthält, wobei die bp126 bis 132 deletiert sind [pHCV-7bp]; pBS/SK-Plasmid mit einem Insert in der EcoRV-Stelle, welches die bp27 bis 313 der cDNA-HCV-Sequenz enthält, wobei ein 8bp langes Insert an der Stelle 126 eingefügt ist [pHCV+8bp] ;

17. pCRII-Plasmid mit einem Insert, welches die bp1763 bis 2032 des HBV enthält [pHBV-wt]; pCRII-Plasmid mit einem Insert, welches die bp1763 bis 2032 des HBV enthält, wobei die bp 1868 bis 1876 deletiert sind [pHBV-9bp] ; pCRII-Plasmid mit einem Insert, welches die bp1763 bis 2032 des HBV enthält, wobei ein 12 bp langes Insert an der Stelle 1868 eingefügt ist [pHBV+12bp] ;

18. pTZ19R-Plasmid mit einem Insert zwischen den beiden PVUII-Stellen, welches die bp83855 bis 84761 des Thymidinkinase-Gens des Vaccinia-Virus enthält, wobei die bp84718 bis 84738 deletiert sind [pVV-21]; pTZ19R-Plasmid mit einem Insert zwischen den beiden PVUII-Stellen dieses Plasmids, welches die bp83855 bis 84761 des Thymidinkinase-Gens des Vaccinia-Virus enthält, wobei ein 24bp langes Insert an der Stelle 84713 eingefügt ist [pVV+24];

19. pCRII-Plasmid mit einem Insert, welches die bp138364 bis 138784 des HSV enthält, wobei die bp138388 bis 138396 deletiert sind [pHerp-9] ;

20. pCRII-Plasmid mit einem Insert, welches die 138364 bis 138784 des HSV enthält, wobei ein 10bp langes Insert an der Stelle 138407 eingefügt ist [pHerp+10].

21. Set zur Quantifizierung von Nukleinsäuren in einer Probe, welches umfaßt:

   - mindestens zwei bekannte Nukleinsäuren als interne Standards, welches sich voneinander und von den zu quantifizierenden Nukleinsäuren in mindestens einem nachweisbaren Charakteristikum unterscheiden,
   - Fluoreszenz-markierte Primer, die an die Standard-Nukleinsäure und an die zu quantifizierende Nukleinsäure binden,
   - positive Kontrollen, welche bekannte Mengen einer Nukleinsäure, an der Interesse besteht, aufweisen,
   - eine negative Kontrolle, welche Humanplasma, das frei von der viralen Nukleinsäure ist, an der Interesse besteht, aufweist, und
   - eine Arbeitsanleitung.

22. Set nach Anspruch 21 zur Quantifizierung von HIV-RNA in einer Probe, welches umfaßt:

   - mindestens zwei interne Standards, die eine in vitro-transkribierte RNA aufweisen, welche vom pBS/SK-Plasmid mit einem Insert in der multiplen Klonierungsstelle, welches die bp1417-2008 des HIV I enthält, wobei die bp1593-1607 deletiert sind, [pgag-15], und vom pBS/SK-Plasmid mit einem Insert in der multiplen Klonierungsstelle, welches die bp1417-2008 des HIV I enthält, wobei ein 12bp langes Insert an der Stelle 1593 eingefügt ist [pgag+12] stammt,
   - die Fluoreszenz-markierten Primer Seq.ID 1 (SK38) und Seq.ID 2 (SK39)
   - eine positive Kontrolle, welche bekannte Mengen an HIV-1-Partikel aufweist,
   - eine negative Kontrolle, welche Humanplasma aufweist, das frei von Virus-Nukleinsäuren ist und
   - eine Arbeitsanleitung.

23. Set nach Anspruch 21 zur Quantifizierung von HCV-RNA in einer Probe, welches aufweist:

   - mindestens zwei interne Standards, die eine in vitro-transkribierte RNA aufweisen, welche vom pBS/SK-Plasmid mit einem Insert in der EcoRV-Stelle, welches die bp27 bis 313 der cDNA-HCV-Sequenz enthält, wobei die bp126 bis 132 deletiert sind, [pHCV-7bp] und vom pBS/SK-Plasmid mit einem Insert in der EcoRV-Stelle, welches die bp27 bis 313 der cDNA-HCV-Sequenz enthält, wobei ein 8bp langes Insert an der Stelle 126 eingefügt ist, [pHCV+8bp] stammt,

- die Fluoreszenz-markierten Primer Seq.ID 5 (HCV32Ext) und Seq.ID 6 (HCVPT4)
- positive Kontrollen, welche bekannte Mengen an HCV-Partikel aufweisen,
- eine negative Kontrolle, welche Humanplasma aufweist, das frei von Virus-Nukleinsäuren ist und
- eine Arbeitsanleitung.

**24.** Set nach Anspruch 21 zur Quantifizierung von HAV-RNA in einer Probe, welches aufweist:

- mindestens zwei interne Standards, die eine in vitro-transkribierte RNA aufweisen, welche vom pCRII-Plasmid mit einem Insert in der multipen Klonierungsstelle, welches die bp2020 bis 2226 der cDNA-HAV-Sequenz enthält, wobei die bp2100 bis 2109 der HAV-Sequenz deletiert sind, [pHAV-10bp] und vom pCRII-Plasmid mit einem Insert in der multiplen Klonierungsstelle, welches die bp2020 bis 2226 der cDNA-HAV-Sequenz enthält, wobei ein 9 bp langes Insert an der Stelle 2100 eingefügt ist, [pHAV+9] stammt,
- die Fluoreszenz-markierten Primer Seq.ID 3 (HAV+2058) und Seq.ID 4 (HAV-2172)
- positive Kontrollen, welche bekannte Mengen an HAV-Partikel aufweisen,
- eine negative Kontrolle, welche Humanplasma aufweist, das frei von Virus-Nukleinsäuren ist und
- eine Arbeitsanleitung.

**25.** Set nach Anspruch 21 zur Quantifizierung von HBV-DNA in einer Probe, welches aufweist:

- mindestens zwei interne Standards, die pCRII-Plasmid mit einem Insert, welches die bp1763 bis 2032 des HBV enthält, wobei die bp 1868 bis 1876 deletiert sind, [pHBV-9bp] und das PCRII-Plasmid mit einem Insert, welches die bp1763 bis 2032 des HBV enthält, wobei ein 12 bp langes Insert an der Stelle 1868 eingefügt ist, [pHBV+12bp] aufweisen,
- die Fluoreszenz-markierten Primer Seq.ID 7 (HBV+1780B) und Seq.ID 8 (HBV-1960B)
- positive Kontrollen, welche bekannte Mengen an HBV-Partikel aufweisen,
- eine negative Kontrolle, welche Humanplasma aufweist, das frei von Virus-Nukleinsäuren ist und
- eine Arbeitsanleitung.

**26.** Set nach Anspruch 21 zur Quantifizierung von HSV-DNA in einer Probe, welches aufweist:

- als interne Standards, die das pCRII-Plasmid mit einem Insert, welches die bp138364 bis 138784 des HSV enthält, wobei die bp138388 bis 138396 deletiert sind [pHerp-9] und das pCRII-Plasmid mit einem Insert, welches die 138364 bis 138784 des HSV enthält, wobei ein 10bp langes Insert an der Stelle 138407 eingefügt ist, [pHerp+10] aufweisen,
- die Fluoreszenz-markierten Primer Seq.ID 11 (gDR1/B) und Seq.ID 12 (gDR2R/B)
- positive Kontrollen, welche bekannte Mengen an HSV-Partikel aufweisen,
- eine negative Kontrolle, welche Humanplasma aufweist, das frei von Virus-Nukleinsäuren ist und
- eine Arbeitsanleitung.

**Claims**

**1.** A method of quantitating nucleic acids in a sample by using nucleic acid amplification, wherein a given amount of a known nucleic acid molecule is added as an internal standard to the sample prior to the amplification step, which standard nucleic acid molecule differs from the nucleic acid to be quantitated in at least one detectable characteristic, **characterised in that** known amounts of at least two known nucleic acid molecules are added as internal standard to the sample prior to the nucleic acid amplification, which at least two known nucleic acid molecules differ from each other and from the nucleic acid to be quantitated by at least one detectable characteristic, the amounts of the amplified sample and standard nucleic acid obtained are determined, and from the obtained amounts, the amount of the nucleic acid to be quantitated originally contained in the sample is determined, and that in the amplification primers containing fluorescent or radioactive groups or chemical groups detectable by affine proteins and subsequent detection reactions are used, preferably with fluorescent groups.

**2.** A method according to claim 1, **characterised in that** the amplification of the nucleic acids is stopped already in the exponential phase.

**3.** A method according to claim 1 or 2, **characterised in that** the determination of the amounts of amplified nucleic acid is effected by using a nucleic acid detection device, preferably a fluorescence-sensitive nucleic acid detection device.

**4.** A method according to any one of claims 1 to 3, **characterised in that** nucleic acids of microorganisms, preferably HIV, Parvo virus, herpes virus, HAV, HBV, HCV, Baculo virus, Adeno virus, vaccinia virus, borrelia species, salmonella species and yeast are quantitated.

**5.** A method according to claim 4, **characterised in that** viral nucleic acids are quantitated in a biological sample, in particular blood, blood derivatives and biotechnological products.

**6.** A method according to any one of claims 1 to 5, **characterised in that** different amounts of the standard nucleic acids are added to the sample prior to amplification.

**7.** A method according to any one of claims 1 to 6, **characterised in that** standard nucleic acids are used which have a length different from that of the nucleic acid to be quantitated, preferably a standard nucleic acid sequence which is shorter, and a standard nucleic acid sequence, which is longer than the nucleic acid to be quantitated.

**8.** A method according to any one of claims 1 to 7, **characterised in that** several obtained amounts of amplified sample and standard nucleic acids are determined in the same sample by means of the multiplex analysis.

**9.** A method according to any one of claims 1 to 8, **characterised in that** the standard nucleic acids are added to the sample before the sample is subjected to one or several sample preparation steps.

**10.** A method of determining the detection limit of certain nucleic acids by using a method according to claims 1 to 9, **characterised in that** at least one standard nucleic acid is used at a concentration slightly exceeding the detection limit.

**11.** The use of a method according to any one of claims 1 to 9 for the detection of nucleic acids in biological samples.

**12.** Primer comprising the sequence according to Seq. ID 3, 4, 5, 7, 8, 9, 10, 11 and 12.

**13.** The use of a primer comprising the sequence according to Seq. ID 6 in a method according to any one of claims 1 to 10.

**14.** pBS/SK plasmid having an insert in the multiple cloning site which contains the bp1417-2008 of HIV I [pgag-1]; pBS/SK-plasmid having an insert in the multiple cloning site, which contains the bp1417-2008 of HIV I, wherein bp1593-1607 are deleted [pgag-15]; bpS/SK plasmid having an insert in the multiple cloning site which contains the bp1417-2008 of HIV I, wherein an insert having a length of 12bp is inserted at site 1593 [pgag+12].

**15.** pCRII-plasmid having an insert in the multiple cloning site which contains the bp2020 to 2226 of the cDNA-HAV sequence [pHAV-wt]; pCRII-plasmid having an insert in the multiple cloning site which contains the bp2020 to 2226 of the cDNA-HAV sequence, wherein the bp2100 to 2109 of the HAV sequence are deleted [pHAV-10bp]; pCRII plasmid having an insert in the multiple cloning site which contains the bp2020 to 2226 of the cDNA-HAV sequence, wherein an insert having a length of 9 bp is inserted at site 2100 [pHAV+9bp].

**16.** pBS/SK plasmid having an insert in the EcoRV site, which contains the bp27 to 313 of the cDNA-HCV sequence [pHCV-wt]; pBS/SK plasmid having an insert in the EcoRV site, which contains the bp27 to 313 of the cDNA-HCV sequence, wherein bp126 to 132 are deleted [pHCV-7bp]; pBS/SK plasmid having an insert in the EcoRV site, which contains the bp27 to 313 of the cDNA-HCV sequence, wherein an insert having a lenth of 8bp is inserted at site 126 [pHCV+8bp].

**17.** pCRII plasmid having an insert which contains the bp1763 to 2032 of HBV [pHBV-wt]; pCRII plasmid having an insert which contains the bp1763-2032 of HBV, wherein bp 1868 to 1876 are deleted [pHBV-9bp]; pCRII plasmid having an insert which contains the bp1763 to 2032 of HBV, wherein an insert having a length of 12 bp is inserted at site 1868 [pHBV+12bp].

**18.** pTZ19R plasmid having an insert between the two PVUII sites, which contains the bp83855 to 84761 of the thymidine kinase gene of the vaccinia virus, wherein bp84718 to 84738 are deleted [pVV-21]; pTZ19R plasmid having an insert between the two PVUII sites of that plasmid, which contains the bp83855 to 84761 of the thymidine kinase gene of the vaccinia virus, wherein an insert having a length of 24 bp is inserted at site 84713 [ppW+24].

19. pCRII plasmid having an insert which contains the bp138364 to 138784 of HSV, wherein bp138388 to 138396 are deleted [pHerp-9] .

20. pCRII plasmid having an insert which contains the pb138364 to 138784 of HSV, wherein an insert having a length of 10 bp is inserted at site 138407 [pHerp+10].

21. A kit for quantitating nucleic acids in a sample comprising

- at least two known nucleic acids as internal standards differing from each other and from the nucleic acids to be quantitated in at least one detectable characteristic,
- fluorescence labelled primers binding to the standard nucleic acid and the nucleic acid to be quantitated,
- positive controls comprising known amounts of a nucleic acid of interest,
- a negative control comprising human plasma free of the viral nucleic acid of interest and
- a manual.

22. A kit according to claim 21 for quantitating HIV-RNA in a sample comprising

- at least two internal standards comprising in vitro transcribed RNA derived from pBS/SK plasmid having an insert in the multiple cloning site, which contains the bp1417-2008 of HIV I, with bp1593-1607 being deleted [pgag-15] and from pBS/SK plasmid having an insert in the multiple cloning site, which contains the bp1417-2008 of HIV I, with an insert having a length of 12bp being inserted at site 1593 [pgag+12],
- the fluorescence labelled primers Seq.ID 1 (SK38) and Seq.ID 2 (SK39)
- a positive control comprising known amounts of HIV I particles
- a negative control comprising human plasma free of viral nucleic acids and
- a manual.

23. A kit according to claim 21 for quantitating HCV-RNA in a sample comprising

- at least two internal standards comprising in vitro transcribed RNA derived from pBS/SK plasmid having an insert in the EcoRV site which contains the bp27 to 313 of the cDNA-HCV sequence, with bp126 to 132 being deleted [pHCV-7] and from pBS/SK plasmid having an insert in the EcoRV site, which contains the bp27 to 313 of the cDNA-HCV sequence, with an insert having a length of 8bp being inserted at site 126 [pHCV+8],
- the fluorescence labelled primers Seq.ID 5 (HCV32Ext) and Seq.ID 6 (HCVPT4)
- positive controls comprising known amounts of HCV particles
- a negative control comprising human plasma free of viral nucleic acids and
- a manual.

24. A kit according to claim 21 for quantitating HAV-RNA in a sample comprising

- at least two internal standards comprising in vitro transcribed RNA derived from pCRII plasmid having an insert in the multiple cloning site, which contains the bp2020 to 2226 of the cDNA-HAV sequence, with bp2100 to 2109 of the HAV sequence being deleted [pHAV-10] and from pCRII plasmid having an insert in the multiple cloning site, which contains the bp2020 to 2226 of the cDNA HAV sequence, with an insert having a length of 9 bp being inserted at site 2100 [pHAV+9],
- the fluorescence labelled primers Seq.ID 3 (HAV+2058) and Seq.ID 4 (HAV-2172)
- positive controls comprising known amounts of HAV particles
- a negative control comprising human plasma free of viral nucleic acids and
- a manual.

25. A kit according to claim 21 for quantitating HBV-DNA in a sample comprising

- at least two internal standards comprising pCRII plasmid having an insert which contains the bp1763 to 2032 of HBV, with bp 1868 to 1876 being deleted [pHBV-9bp] and the PCRII plasmid having an insert which contains the bp1763 to 2032 of HBV, with an insert having a length of 12 bp being inserted at site 1868 [pHBV+12bp],
- the fluorescence labelled primers Seq.ID 7 (HBV+1780B) and Seq.ID 8 (HBV-1960B)
- positive controls comprising known amounts of HBV particles

- a negative control comprising human plasma free of viral nucleic acids and
- a manual.

**26.** A kit according to claim 21 for quantitating HSV-DNA in a sample comprising

- as internal standards, the pCRII plasmid having an insert containing the bp138364 to 138784 of HSV, with bp138388 to 138396 being deleted [pHerp-9] and the pCRII plasmid having an insert which contains the bp138364 to 138784 of HSV, with an insert having a length of 10 bp being inserted at site 138407 [pHerp+10),
- the fluorescence labelled primers Seq.ID 11 (gDR1/B) and Seq.ID 12 (gDR2R/B)
- positive controls comprising known amounts of HSV particles
- a negative control comprising human plasma free of viral nucleic acids and
- a manual.

**Revendications**

**1.** Procédé de quantification d'acides nucléiques dans un échantillon, par utilisation d'une amplification d'acides nucléiques, dans lequel on ajoute à l'échantillon, avant l'étape d'amplification, une quantité donnée d'une molécule connue d'acide nucléique servant d'étalon interne, laquelle molécule d'acide nucléique étalon se distingue de l'acide nucléique à quantifier par au moins une caractéristique détectable, **caractérisé en ce qu'**on ajoute à l'échantillon, en tant qu'étalon interne, avant amplification de l'acide nucléique, des quantités connues d'au moins deux molécules d'acide nucléique connues, qui se distinguent par au moins une caractéristique détectable l'une de l'autre et de l'acide nucléique à quantifier, on détermine les quantités obtenues d'acide nucléique amplifié, échantillon et étalon, et, à partir des quantités obtenues, on détermine la quantité initialement présente dans l'échantillon de l'acide nucléique à quantifier, et **en ce que**, lors de l'amplification, on utilise des amorces comportant des groupes fluorescents ou radioactifs ou des groupes chimiques, qui peuvent être détectés par des protéines affines puis par des réactions de détection effectuées ensuite, de préférence des groupes fluorescents.

**2.** Procédé selon la revendication 1, **caractérisé en ce que** l'amplification des acides nucléiques est interrompue déjà dans la phase exponentielle.

**3.** Procédé selon la revendication 1 ou 2, **caractérisé en ce que** la détermination des quantités d'acide nucléique amplifié s'effectue par utilisation d'un appareil de détection des acides nucléiques, de préférence un appareil de détection des acides nucléiques sensible à la fluorescence.

**4.** Procédé selon l'une des revendications 1 à 3, **caractérisé en ce que** l'on quantifie des acides nucléiques de microorganismes, de préférence le VIH, les parvovirus, les herpesvirus, le HAV, le HBV, le HCV, les baculovirus, les adénovirus, les virus vaccinaux, les espèces de Borrelia, les espèces de Salmonella et la levure.

**5.** Procédé selon la revendication 4, **caractérisé en ce que** l'on quantifie des acides nucléiques viraux dans un échantillon biologique, en particulier de sang et de dérivés du sang, et des produits biologiques.

**6.** Procédé selon l'une des revendications 1 à 5, **caractérisé en ce qu'**on ajoute à l'échantillon, avant amplification, des quantités différentes des acides nucléiques étalons.

**7.** Procédé selon l'une des revendications 1 à 6, **caractérisé en ce qu'**on utilise des acides nucléiques étalons qui présentent une taille différente de celle de l'acide nucléique à quantifier, de préférence une séquence d'acide nucléique étalon qui est plus courte, et une séquence d'acide nucléique étalon qui est plus longue que l'acide nucléique à quantifier.

**8.** Procédé selon l'une des revendications 1 à 7, **caractérisé en ce que** plusieurs quantités déjà obtenues d'acides nucléiques échantillons et étalons amplifiés sont déterminées dans le même échantillon à l'aide d'une analyse multiplex.

**9.** Procédé selon l'une des revendications 1 à 8, **caractérisé en ce que** les acides nucléiques étalons sont ajoutés à l'échantillon, encore avant que l'échantillon ne soit soumis à une ou plusieurs étapes de préparation de l'échantillon.

**10.** Procédé pour déterminer le seuil de détection de certains acides nucléiques, par utilisation d'un procédé selon la revendication 1 à 9, **caractérisé en ce qu'**on utilise au moins un acide nucléique étalon, à une concentration juste supérieure à la limite de détection.

**11.** Utilisation d'un procédé selon l'une des revendications 1 à 9 pour détecter des acides nucléiques dans des échantillons biologiques.

**12.** Amorce dont la séquence correspond aux séquences Seq.ID 3, 4, 5, 7, 8, 9, 10, 11 et 12.

**13.** Utilisation d'une amorce dont la séquence correspond à la séquence Seq.ID 6 dans un procédé selon l'une des revendications 1 à 10.

**14.** Plasmide pBS/SK, ayant un insert sur la position de clonage multiple qui contient les pb1417-2008 du VIH I [pgag-1] ; plasmide pBS/SK ayant un insert sur la position de clonage multiple qui contient les pb1417-2008 du VIH I, où les pb1593-1607 sont supprimés [pgag-15] ; plasmide pbS/SK ayant un insert sur la position de clonage multiple qui contient les pb1417-2008 du VIH I, où un insert de 12pb est inséré sur la position 1593 [pgag+12].

**15.** Plasmide pCRII ayant un insert sur la position de clonage multiple qui contient les pb2020 à 2226 de la séquence de l'ADNc-HAV [pHAV-wt] ; plasmide pCRII ayant un insert sur la position de clonage multiple qui contient la séquence pb2020 à 2226 de l'ADNc-HAV, où les pb2100 à 2109 de la séquence du HAV sont supprimées [pHAV-10pb] ; plasmide pCRII ayant un insert sur la position de clonage multiple, qui contient les pb2020 à 2226 de la séquence de l'ADNc-HAV, où un insert de 9pb est inséré sur la position 2100 [pHAV+9pb].

**16.** Plasmide pBS/SK ayant un insert sur la position EcoRV qui contient les pb27 à 313 de la séquence de l'ADNc-HCV [pHCV-wt] ; plasmide pBS/SK ayant un insert sur la position EcoRV qui contient les pb27 à 313 de la séquence de l'ADNc-HCV, où les pb126 à 132 sont supprimés [pHCV-7pb] ; plasmide pBS/SK ayant un insert sur la position EcoRV qui contient les pb27 à 313 de la séquence de l'ADNc-HCV, où un insert de 8pb est inséré sur la position 126 [pHCV+8pb].

**17.** Plasmide pCRII ayant un insert qui contient les pb1763 à 2032 du HBV [pHBV-wt] ; plasmide pCRII ayant un insert qui contient les pb1763 à 2032 du HBV, où les pb 1868 à 1876 sont supprimés [pHBV-9pb] ; plasmide pCRII ayant un insert qui contient les pb1763 à 2032 du HBV, où un insert de 12 pb est inséré sur la position 1868 [pHBV+12pb].

**18.** Plasmide pTZ19R ayant un insert entre les deux positions PVUII qui contient les pb83855 à 84761 du gène de la thymidinekinase du virus vaccinal, où les pb84718 à 84738 sont supprimés [pVV-21] ; plasmide pTZ19R ayant un insert compris entre les deux positions PVUII de ce plasmide, qui contient les pb83855 à 84761 du gène de la thymidinekinase du virus vaccinal, où un insert de 24pb est inséré sur la position 84713 [pVV+24].

**19.** Plasmide pCRII, ayant un insert qui contient les pb138364 à 138784 du HSV, où les pb138388 à 138396 sont supprimés [pHerp-9].

**20.** Plasmide pCRII ayant un insert qui contient les pb138364 à 138784 du HSV, où un insert de 10pb est inséré sur la position 138407 [pHerp+10].

**21.** Trousse pour quantifier des acides nucléiques dans un échantillon, qui comprend :

- au moins deux acides nucléiques connus servant d'étalons internes, qui se distinguent l'un de l'autre et des acides nucléiques à quantifier par au moins une caractéristique détectable,
- des amorces marquées par fluorescence, qui se lient à l'acide nucléique étalon et à l'acide nucléique à quantifier,
- des témoins positifs, qui contiennent des quantités connues d'un acide nucléique présentant un intérêt,
- un témoin négatif, qui contient du plasma humain exempt de l'acide nucléique viral présentant un intérêt, et
- un mode d'emploi.

**22.** Trousse selon la revendication 21 pour quantifier l'ARN du VIH dans un échantillon, qui comporte

- au moins deux étalons internes, qui comportent un ARN transcrit in vitro, lequel provient du plasmide pBS/SK ayant un insert sur la position de clonage multiple qui contient les pb1417-2008 du VIH I, où les pb1593-1607 sont supprimés [pgag-15], et du plasmide pBS/SK, ayant un insert sur la position de clonage multiple qui contient les pb1417-2008 du VIH I, où un insert de 12pb est inséré sur la position 1593 [pgag+12],
- les amorces marquées par fluorescence Seq.ID 1 (SK38) et Seq.ID 2 (SK39),
- un témoin positif qui contient des quantités connues de particules de VIH-1,
- un témoin négatif qui contient du plasma humain exempt d'acides nucléiques viraux, et
- un mode d'emploi.

**23.** Trousse selon la revendication 21 pour quantifier l'ARN du HCV dans un échantillon, qui comporte

- au moins deux étalons internes, qui comportent un ARN transcrit in vitro, qui dérive du plasmide pBS/SK ayant un insert sur la position EcoRV qui contient les pb27 à 313 de la séquence de l'ADNc/HCV, où les pb126 à 132 sont supprimés [pHCV-7pb], et du plasmide pBS/SK, ayant un insert sur la position EcoRV qui contient les pb27 à 313 de la séquence de l'ADNc-HCV, où un insert de 8pb est inséré sur la position 126 [pHCV+8pb],
- les amorces marquées par fluorescence Seq.ID 5 (HCV32Ext) et Seq.ID 6 (HCVPT4),
- des témoins positifs qui contiennent des quantités connues de particules de HCV,
- un témoin négatif qui contient un plasma humain exempt d'acides nucléiques viraux, et
- un mode d'emploi.

**24.** Trousse selon la revendication 21 pour quantifier l'ARN du HAV dans un échantillon, qui contient

- au moins deux étalons internes, qui contiennent un ADN transcrit in vitro, qui dérivent du plasmide pCRII, ayant un insert sur la position de clonage multiple qui contient les pb2020 à 2226 de la séquence de l'ADNc-HAV, où les pb2100 à 2109 de la séquence du HAV sont supprimés [pHAV-10pb] et du plasmide pCRII, ayant un insert sur la position de clonage multiple qui contient les pb2020 à 2226 de la séquence de l'ADNc-HAV, où un insert de 9 pb est inséré sur la position 2100 [pHAV+9],
- les amorces marquées par fluorescence Seq.ID 3 [HAV+2058], et Seq.ID 4 [HAV-2172),
- des témoins positifs contenant des quantités connues de particules de HAV,
- un témoin négatif qui contient un plasma humain exempt d'acides nucléiques viraux, et
- un mode d'emploi.

**25.** Trousse selon la revendication 21 pour quantifier l'ADN du HBV dans un échantillon, qui contient

- au moins deux étalons internes qui contiennent le plasmide pCRII, ayant un insert qui contient les pb1763 à 2032 du HBV, où les pb1868 à 1876 sont supprimés [pHBV-9pb], et le plasmide PCRII, ayant un insert qui contient les pb1763 à 2032 du HBV, où un insert de 12 pb est inséré sur la position 1868 [pHBV+12pb],
- les amorces marquées par fluorescence Seq.ID 7 (HBV+1780B) et Seq.ID 8 (HBV-1960B),
- des témoins positifs, qui contiennent des quantités connues de particules de HBV,
- un témoin négatif, qui contient un plasma humain exempt d'acides nucléiques viraux, et
- un mode d'emploi.

**26.** Trousse selon la revendication 21 pour quantifier l'ADN du HSV dans un échantillon, qui contient

- en tant qu'étalons internes, des étalons internes qui contiennent le plasmide pCRII, ayant un insert qui contient les pb138364 à 138784 du HSV, où les pb138388 à 138396 sont supprimés [pHerp-9], et le plasmide pCRII, ayant un insert qui contient les pb138364 à 138784 du HSV, où un insert de 10 pb est inséré sur la position 138407 [pHERp+10],
- les amorces marquées par fluorescence Seq.ID 11 (gDR1/B) et Seq.ID 12 (gDR2R/B),
- des témoins positifs contenant des quantités connues de particules de HSV,
- un témoin négatif qui contient un plasma humain exempt d'acides nucléiques viraux, et
- un mode d'emploi.

Fig.1

Fig.2

Fig.3-A

Fig.3-B

Fig.4-A

Fig.4-B

Fig.4-C

Fig.4-D

Fig.4-E

SEQUENZPROTOKOLL

(1) ALLGEMEINE ANGABEN:

    (i) ANMELDER:
        (A) NAME: Falko-Guenther Falkner
        (B) STRASSE: Neusiedlzeile 76A
        (C) ORT: Orth/Donau
        (D) BUNDESLAND: Austria
        (E) LAND: Austria
        (F) POSTLEITZAHL: 2304

        (A) NAME: Thomas Haemmerle
        (B) STRASSE: Hauptstrasse 46
        (C) ORT: Orth/Donau
        (D) BUNDESLAND: Austria
        (E) LAND: Austria
        (F) POSTLEITZAHL: 2304

        (A) NAME: Michele Himmelspach
        (B) STRASSE: Laxenburgerstrasse 59/13
        (C) ORT: Wien
        (D) BUNDESLAND: Austria
        (E) LAND: Austria
        (F) POSTLEITZAHL: 1100

        (A) NAME: Johann Kohl
        (B) STRASSE: Schuhmeierplatz 3/8
        (C) ORT: Wien
        (D) BUNDESLAND: Austria
        (E) LAND: Austria
        (F) POSTLEITZAHL: 1160

        (A) NAME: Friedrich Dorner
        (B) STRASSE: Peterlinigasse 17
        (C) ORT: Wien
        (D) BUNDESLAND: Austria
        (E) LAND: Austria
        (F) POSTLEITZAHL: 1238

    (ii) BEZEICHNUNG DER ERFINDUNG: Verfahren zur Quantifizierung von
        Nukleinsaeuren

    (iii) ANZAHL DER SEQUENZEN: 12

    (iv) COMPUTER-LESBARE FASSUNG:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30 (EPA)

(2) ANGABEN ZU SEQ ID NO: 1:

    (i) SEQUENZKENNZEICHEN:
        (A) LÄNGE: 28 Basenpaare
        (B) ART: Nucleotid
        (C) STRANGFORM: Einzelstrang
        (D) TOPOLOGIE: linear

    (ii) ART DES MOLEKÜLS: Genom-DNA

    (xi) SEQUENZBESCHREIBUNG: SEQ ID NO: 1:

ATAATCCACC TATCCCAGTA GGAGAAAT         28

# Fig.5-A

41

(2) ANGABEN ZU SEQ ID NO: 2:

    (i) SEQUENZKENNZEICHEN:
       (A) LÄNGE: 28 Basenpaare
       (B) ART: Nucleotid
       (C) STRANGFORM: Einzelstrang
       (D) TOPOLOGIE: linear

    (ii) ART DES MOLEKÜLS: Genom-DNA

    (xi) SEQUENZBESCHREIBUNG: SEQ ID NO: 2:

TTTGGTCCTT GTCTTATGTC CAGAATGC                    28

(2) ANGABEN ZU SEQ ID NO: 3:

    (i) SEQUENZKENNZEICHEN:
       (A) LÄNGE: 24 Basenpaare
       (B) ART: Nucleotid
       (C) STRANGFORM: Einzelstrang
       (D) TOPOLOGIE: linear

    (ii) ART DES MOLEKÜLS: Genom-DNA

    (xi) SEQUENZBESCHREIBUNG: SEQ ID NO: 3:

ACTGCCATTG GGAAGCTTAT TGTG                        24

(2) ANGABEN ZU SEQ ID NO: 4:

    (i) SEQUENZKENNZEICHEN:
       (A) LÄNGE: 25 Basenpaare
       (B) ART: Nucleotid
       (C) STRANGFORM: Einzelstrang
       (D) TOPOLOGIE: linear

    (ii) ART DES MOLEKÜLS: Genom-DNA

    (xi) SEQUENZBESCHREIBUNG: SEQ ID NO: 4:

CATCCATAGC ATGATAAAGA GGAGC                      25

(2) ANGABEN ZU SEQ ID NO: 5:

    (i) SEQUENZKENNZEICHEN:
       (A) LÄNGE: 26 Basenpaare
       (B) ART: Nucleotid
       (C) STRANGFORM: Einzelstrang
       (D) TOPOLOGIE: linear

    (ii) ART DES MOLEKÜLS: Genom-DNA

    (xi) SEQUENZBESCHREIBUNG: SEQ ID NO: 5:

CTGTGAGGAA CTACTGTCTT ACGCAG                 26

## Fig.5-B

(2) ANGABEN ZU SEQ ID NO: 6:

    (i) SEQUENZKENNZEICHEN:
        (A) LÄNGE: 20 Basenpaare
        (B) ART: Nucleotid
        (C) STRANGFORM: Einzelstrang
        (D) TOPOLOGIE: linear

    (ii) ART DES MOLEKÜLS: Genom-DNA


    (xi) SEQUENZBESCHREIBUNG: SEQ ID NO: 6:

CGGTTCCGCA GACCACTATG                                            20


(2) ANGABEN ZU SEQ ID NO: 7:

    (i) SEQUENZKENNZEICHEN:
        (A) LÄNGE: 27 Basenpaare
        (B) ART: Nucleotid
        (C) STRANGFORM: Einzelstrang
        (D) TOPOLOGIE: linear

    (ii) ART DES MOLEKÜLS: Genom-DNA


    (xi) SEQUENZBESCHREIBUNG: SEQ ID NO: 7:

CATTGATCCT TATAAAGAAT TTGGAGC                              27


(2) ANGABEN ZU SEQ ID NO: 8:

    (i) SEQUENZKENNZEICHEN:
        (A) LÄNGE: 24 Basenpaare
        (B) ART: Nucleotid
        (C) STRANGFORM: Einzelstrang
        (D) TOPOLOGIE: linear

    (ii) ART DES MOLEKÜLS: Genom-DNA


    (xi) SEQUENZBESCHREIBUNG: SEQ ID NO: 8:

CCAGCAGAGA ATTGCTTGCC TGAG                                    24


(2) ANGABEN ZU SEQ ID NO: 9:

    (i) SEQUENZKENNZEICHEN:
        (A) LÄNGE: 19 Basenpaare
        (B) ART: Nucleotid
        (C) STRANGFORM: Einzelstrang
        (D) TOPOLOGIE: linear

    (ii) ART DES MOLEKÜLS: Genom-DNA


    (xi) SEQUENZBESCHREIBUNG: SEQ ID NO: 9:

AAAATAGGAT CATGATGGC                                             19

Fig. 5-C

(2) ANGABEN ZU SEQ ID NO: 10:

   (i) SEQUENZKENNZEICHEN:
     (A) LÄNGE: 20 Basenpaare
     (B) ART: Nucleotid
     (C) STRANGFORM: Einzelstrang
     (D) TOPOLOGIE: linear

   (ii) ART DES MOLEKÜLS: Genom-DNA

   (xi) SEQUENZBESCHREIBUNG: SEQ ID NO: 10:

ATATTAGATG GTGCCACCGT                 20


(2) ANGABEN ZU SEQ ID NO: 11:

   (i) SEQUENZKENNZEICHEN:
     (A) LÄNGE: 18 Basenpaare
     (B) ART: Nucleotid
     (C) STRANGFORM: Einzelstrang
     (D) TOPOLOGIE: linear

   (ii) ART DES MOLEKÜLS: Genom-DNA

   (xi) SEQUENZBESCHREIBUNG: SEQ ID NO: 11:

AACTACCCCG ATCATCAG                 18


(2) ANGABEN ZU SEQ ID NO: 12:

   (i) SEQUENZKENNZEICHEN:
     (A) LÄNGE: 18 Basenpaare
     (B) ART: Nucleotid
     (C) STRANGFORM: Einzelstrang
     (D) TOPOLOGIE: linear

   (ii) ART DES MOLEKÜLS: Genom-DNA

   (xi) SEQUENZBESCHREIBUNG: SEQ ID NO: 12:

AGGCCCACTA TGACGACA                 18


# Fig.5-D